# EUROPEAN PATENT APPLICATION

(11) **EP 4 155 404 A1**
(43) Date of publication of application: **29.03.2023**
(21) Application number: 21833239.3
(22) Date of filing: 29.06.2021
(51) Int. Cl.: C12N 15/115, A61K 47/18

(54) **BLOOD-BRAIN BARRIER PENETRATING APTAMER, AND USE THEREOF**

(30) Priority: 29.06.2020 KR 20200079495; 24.06.2021 KR 20210082374
(71) Applicant: Aptamer Sciences Inc., Seongnam-si, Gyeonggi-do 13605 (KR)
(72) Inventor: KIM, Youndong, Seongnam-si, Gyeonggi-do 13602 (KR); LEE, Da Som, Seoul 08213 (KR); KIM, Soyeon, Seoul 06096 (KR); LIM, So Ryong, Yongin-si, Gyeonggi-do 16963 (KR); SHIN, Euisu, Suwon-si, Gyeonggi-do 16686 (KR); KANG, Naye, Gwangju-si, Gyeonggi-do 12820 (KR); HAN, Dongil, Yongin-si, Gyeonggi-do 16849 (KR)
(74) Representative: Gassner, Birgitta
(86) International application number: PCT/KR2021/008212
(87) International publication number: WO 2022/005179

(57) **Abstract**

The present invention relates to an aptamer penetrating the blood-brain barrier (BBB) to enable receptor-mediated transcytosis and a use thereof.

The aptamer of the present invention selectively targets transferring receptor proteins only, rapidly passes through BBB by means of receptor-mediated transcytosis, can effectively transport and deliver various therapeutic agents such as proteins, antibodies, nucleic acids, and low-molecular weight compounds through simple chemical bonds, exhibits interspecific cross-reactivity in transferring receptors, and is easily chemically modified, and thus is applicable as a drug carrier for nervous system diseases, especially brain and central nervous systems.

## Description

### Technical Field

The present invention relates to an aptamer penetrating the blood-brain barrier (BBB) of humans and mice by means of receptor-mediated transcytosis and a use thereof.

### Background Art

Aptamers are single-stranded nucleic acids (DNA, RNA, or modified nucleic acids) having a stable three-dimensional structure and a property of binding to target molecules with high affinity and specificity. Since systematic evolution of ligands by exponential enrichment (SELEX) was first developed in 1990 as a method of developing aptamers, many aptamers capable of binding to various target molecules, such as low-molecular weight organic materials, peptides, and membrane proteins, have been developed. Due to intrinsic high binding affinity (normal pM level) and the ability to specifically bind to target molecules, aptamers have often been compared with single antibodies and have a high likelihood of acting as antibody substitutes to the extent that aptamers are also called "chemical antibodies".

As aptamers binding to various types of target molecules have been reported, continuous efforts have been made to use aptamers, binding to target molecules mediated in various diseases, as therapeutic agents, and particularly, if developed, aptamers serving as antagonists for molecules causing diseases have been expected to be used in targeted therapy. As a result, Macugen, which is an aptamer drug co-developed by Pfizer Inc. and Eyetech Pharmaceuticals Inc., was approved by the FDA in late 2004 and marketed. Macugen blocks binding of the VEGF165 protein to a VEGF receptor by specifically binding to VEGF165 protein that causes age-related macular degeneration (AMD), thereby having excellent therapeutic effects on AMD. Archemix Corp. has developed various new drug candidates such as ARC1779 (for treatment of thrombotic microvascular diseases) which suppresses thrombosis by selectively binding to vWF causing excessive thrombosis. Also, intensive research has been conducted on aptamer drugs for virus-mediated diseases, and since an aptamer binding to T4 DNA polymerase has first been reported, many aptamers for proteins produced by HIV-1 and HCV have been developed.

Meanwhile, with the increase in the number of nervous system diseases such as brain cancer, brain infection, and CNS disease, the demands on advanced technologies for drug delivery are increasing. However, a drug cannot be delivered to the brain by administering the drug to blood due to blood-brain barrier (BBB). The blood-brain barrier, as a blood vessel barrier isolating the brain from blood, has highly selective permeability and serves to separate the regulatory function of the central nervous system including the brain from pathogens such as bacteria that may be transported via blood and from potentially dangerous substances contained in the blood. Endothelial cells of brain capillary form tight junctions by substances secreted from pacemakers of astrocytes to interfere with intercellular migration of solutes, thereby blocking passage of polymers and hydrophilic substances therethrough. Therefore, particular channels or transporter proteins are required for penetration of water-soluble molecules through the blood-brain barrier.

### Disclosure

### Technical Problem

The present inventors have developed an aptamer penetrating the blood-brain barrier of both humans and mice by means of receptor-mediated transcytosis, thereby completing the present invention.

### Technical Solution

An object of the present invention is to provide a transferrin receptor protein-specific aptamer.

Another object of the present invention is to provide a pharmaceutical composition for preventing or treating a nervous system disease including the aptamer as an active ingredient.

Another object of the present invention is to provide a drug carrier including the aptamer.

### Advantageous Effects

The aptamer of the present invention may be applicable as a drug carrier for nervous system diseases, particularly brain and central nervous system diseases, because the aptamer selectively targets the transferrin receptor proteins only, rapidly passes through the BBB by means of receptor-mediated endocytosis, may efficiently transport and deliver various therapeutic agents such as proteins, antibodies, nucleic acids, and low-molecular weight compounds through simple chemical bonds, exhibits interspecific cross-reactivity in transferrin receptors, and is easily chemically modified.

### Brief Description of Drawings

FIG. 1 is a diagram showing transferrin receptor protein binding ability of TfR aptamers.
FIG. 2 is a diagram showing cell binding ability of TfR aptamers.
FIG. 3 is a diagram showing human and mouse cell line binding ability of TfR aptamers.
FIG. 4 is a diagram showing receptor-mediated endocytosis of mouse cell lines by TfR aptamers.
FIG. 5 is a diagram showing receptor-mediated endocytosis of human and mouse cell lines by GFP-conjugated TfR aptamers.
FIG. 6 is a diagram showing receptor-mediated endocytosis of human, monkey, mouse cell lines by Cetuximab- or Rituximab-conjugated TfR aptamers.
FIG. 7 is a schematic diagram illustrating an *in vitro* brain microvascular structure.
FIG. 8 is a diagram showing effects of a TfR aptamer on penetrating the *in vitro* brain microvascular structure.
FIG. 9 is a diagram showing distribution of an antibody delivered into the brain by a TfR aptamer.
FIG. 10 is a diagram showing distribution of an antibody delivered into the brain by a Cetuximab-conjugated aptamer.
FIG. 11 is a diagram showing distribution of an antibody delivered into the brain by a Rituximab-conjugated aptamer.
FIG. 12 is a diagram showing effects of an antibody-conjugated TfR aptamer on circulating reticulocytes.
FIG. 13 is a diagram showing transferrin receptor protein binding ability of chemically optimized TfR aptamers.
FIG. 14 is a diagram showing receptor-mediated endocytosis of human, mouse, and monkey cell lines by chemically optimized TfR aptamers.
FIG. 15 is a diagram showing serum stability of a chemically optimized TfR aptamers.
FIG. 16 is a diagram showing receptor-mediated endocytosis of an antibody-conjugated chemically optimized TfR aptamer.
FIG. 17 is a diagram showing transferrin receptor protein binding ability of a TfR aptamer according to pH conditions.

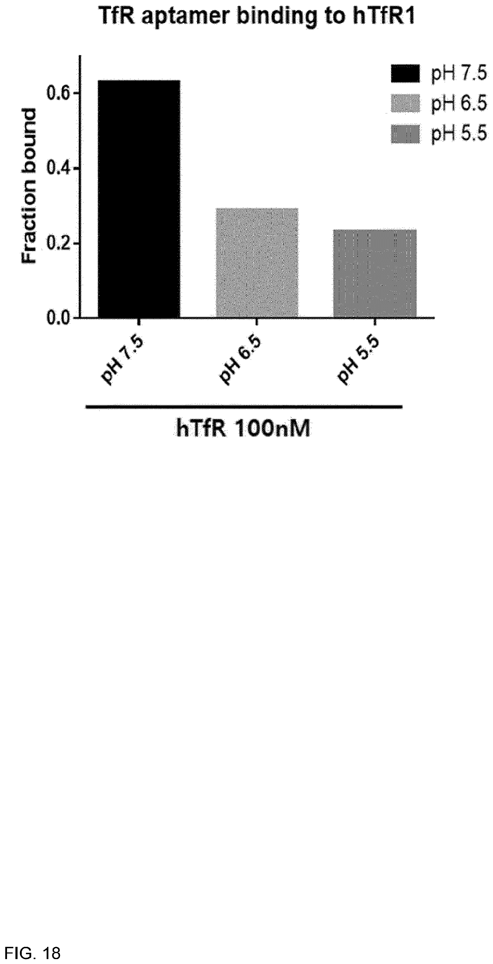

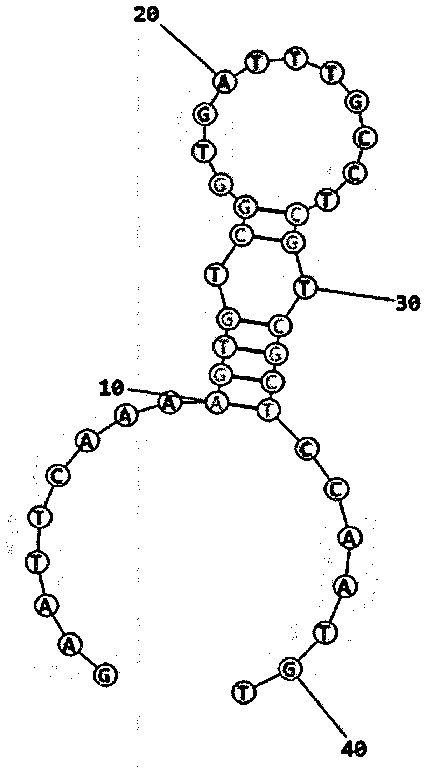

FIG. 18 is a diagram illustrating a structure of a TfR aptamer of the present invention (TfR-01-01-41, SEQ ID NO: 1).

### Best Mode

Hereinafter, the present invention will be described in detail. Meanwhile, each of the descriptions and embodiments disclosed herein may be applied herein to describe different descriptions and embodiments. That is, all of the combinations of various factors disclosed herein belong to the scope of the present invention. Furthermore, the scope of the present invention should not be limited by the detailed descriptions provided hereinbelow.

To solve the above-described problems, an aspect of the present invention provides a transferrin receptor protein-specific aptamer.

The aptamer of the present invention may have a nucleotide sequence of General Formula 1 below:

[General Formula 1] AGTGTCGGTGATTTGCCTCGTCGCT (SEQ ID NO: 52).

As used herein, the term "transferrin receptor" refers to a protein transporting iron into cells in response to intracellular iron concentration. The transferrin receptor is known to import iron into a cell by internalizing a transferrin-iron complex by means of receptor-mediated endocytosis and may pass through the blood-brain barrier (BBB) by means of the receptor-mediated endocytosis.

As used herein, the term "aptamer" refers to a single-stranded nucleic acid molecule, as a "chemical antibody", having a short sequence (20 to 80 nucleotides) with the ability to bind to various types of target ligands such as particular compounds and proteins with high specificity and affinity. Aptamers may be developed *in vitro* by systematic evolution of ligands by exponential enrichment (SELEX).

Aptamers are considered as oligo-nucleic acid molecules having similar properties to those of antibodies in terms of high binding ability and selectivity to target proteins at the femtomole (fM) to nanomole (nM) level. Meanwhile, when compared with antibodies, aptamers have various advantages as follows: (1) Aptamers prepared by chemical synthesis may be chemically modified more easily than protein-based substances such as antibodies, (2) Selectivity and affinity may be maximized by a SELEX process, (3) High purity may be obtained as aptamers are made by chemical synthesis, (4) Substances may be identified by instrumental analysis, and (5) Long-term storage at room temperature is possible due to high thermal stability.

As used herein, the term "systematic evolution of ligands by exponential enrichment (SELEX)" refers to a method for selecting an aptamer binding to a target substance. In the case of methods commonly known in the art, after reaction between a target protein and an oligonucleotide library (DNA or RNA) consisting of random nucleotide sequences at a certain temperature, unbound DNAs/RNAs are removed. After separating nucleotides binding to the target, the nucleotides are amplified by gene amplification, and this process is repeated several times to select an aptamer having a high binding ability to the target.

The aptamer of the present invention may be prepared by using a common SELEX method as described above.

In an aptamer selection process, a process of obtaining a pool of single-stranded nucleic acids from a library including about 10¹⁴ to 10¹⁵ different sequences, *i.e.,* having diversity, is generally required. Although various methods have been used therefor, in general, a method of amplifying a single-stranded nucleic acid only by asymmetric PCR and a method of selectively separating a single-strand of a doublestranded nucleic acid using streptavidin-coated beads after biotinylating one terminus of the single-strand have been widely used.

Then, a process of selecting an aptamer having high binding ability to a target molecule is performed by binding the obtained library to the target molecule. When the target molecule is a protein, biotin labeled at the protein is pulled down using streptavidin beads. After inducing binding of the target protein to a modified nucleic acid library, the resultant is rinsed with a buffer to remove unbound nucleic acids of the modified nucleic acid library.

Similarly, in the case of plate, after inducing binding of the target protein to a nucleic acid library, the resultant is rinsed with a buffer to remove unbound nucleic acids. By using these methods, aptamers having affinity to a ligand may be obtained. In general, aptamers having high affinity may be obtained by repeating a selection-amplification process 5 to 15 times. Upon completion of the selection process, amplified nucleic acids are cloned and sequenced from each of the clones, and then aptamers are synthesized and affinity and binding ability of the aptamers to the target molecule are measured.

As used herein, the term "target molecule" refers to a substance detectable by the aptamer of the present invention. Specifically, the target molecule may include at least one selected from the group consisting of protein, peptide, carbohydrate, polysaccharide, glycoprotein, hormone, receptor, antigen, antibody, virus, cofactor, drug, dye, growth factor, and controlled substance which are present in a separated sample and able to bind to a capture aptamer, without being limited thereto. In view of the objects of the present invention, the target molecule may be a transferrin receptor protein.

The aptamer of the present invention may further include 1 to 30 polynucleotides bound to at least one of the 5'-terminus and 3'-terminus.

The aptamer may include 30 to 150 polynucleotides by further including the above-described polynucleotides bound to at least one of the 5'-terminus and 3'-terminus thereof.

The aptamer of the present invention may include at least one nucleotide sequence selected from the group consisting of SEQ ID NOS: 1 to 4 or a nucleotide sequence having at least 80%, 85%, 90%, 95%, 96%, 97%, 98%, or 99% homology or identity therewith. Also, it is obvious that any polynucleotide having a nucleotide sequence including deletion, modification, substitution, or addition of one or several nucleotides may also be included in the present invention as long as the polynucleotide has such homology or identity and effects equivalent to those of the aptamer.

As used herein, the term "homology" or "identity" refers to relatedness between two amino acid sequences or nucleotide sequences and may be expressed as a percentage. The homology and identity may often be used interchangeably.

Sequence homology or identity of conserved polynucleotides or polypeptides may be determined by standard alignment algorithm and default gap penalties established by a program may be used together therewith. Substantially, homologous or identical sequences may generally hybridize with each other in whole or by at least about 50%, 60%, 70%, 80%, or 90% of the entire sequence under moderately or highly stringent conditions. Polynucleotides including degenerated codon instead of codon may also be considered in hybridization.

Homology, similarity, or identity between two polynucleotide or polypeptide sequences may be determined using any computer algorithm known in the art, *e.g.,* "FASTA" program, using default parameters introduced by Pearson et al (1988) Proc. Natl. Acad. Sci. USA 85:2444. Alternatively, the homology, similarity, or identity may be determined using the Needleman-Wunsch algorithm (Needleman and Wunsch, 1970, J. Mol. Biol. 48:443-453) as implemented in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST, from the National Center for Biotechnology Information database, or ClustalW.

The homology, similarity, or identity between polynucleotides or polypeptides may be determined by comparing sequence information using a GAP computer program as introduced by Needleman et al., (1970), J Mol Biol. 48:443 as disclosed by Smith and Waterman, Adv. Appl. Math (1981) 2:482. Briefly, the GAP program defines similarity as the number of aligned symbols (*i.e.,* nucleotides or amino acids) which are similar, divided by the total number of symbols in a shorter of two sequences. Default parameters for the GAP program may include: (1) a binary comparison matrix (containing a value of 1 for identities and 0 for non-identities) and the weighted comparison matrix of Gribskov et al. (1986), Nucl. Acids Res. 14:6745 as described by Schwartz and Dayhoff, eds., Atlas Of Protein Sequence and Structure, National Biomedical Research Foundation, pp. 353-358 (1979) (or EDNAFULL (EMBOSS version of NCBI NUC4.4) substitution matrix); (2) a penalty of 3.0 for each gap and an additional penalty of 0.10 for each symbol in each gap (or a gap open penalty of 10 and a gap extension penalty of 0.5); and (3) no penalty for end gap.

Also, the sequence homology, similarity, or identity between two polynucleotide or polypeptide sequences may be identified by comparing sequences thereof by southern hybridization under defined stringent conditions, and the defined stringent hybridization conditions are within the scope of the technology and may be defined by a method well known to one of ordinary skill in the art (*e.g.,* J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York).

The aptamer may include at least one modification, selected from the following modifications i) to iii) at one or more nucleotides constituting a nucleotide sequence thereof:
i) 5^{th} carbon of thymine (T) of at least one nucleotide is substituted with at least one selected from the group consisting of a benzyl group, a naphthyl group, a 2-naphthyl group, a pyrrolebenzyl group, and tryptophan;
ii) -OH group of the 2^{nd} carbon of at least one nucleotide is substituted with at least one selected from the group consisting of a deoxy group, a methoxy group, an amino group, and fluorine (F); and
iii) at least one nucleotide is substituted with at least one selected from the group consisting of methylphosphonate, phosphorothioate, locked nucleic acid (LNA), peptide nucleic acid (PNA), and hexitol nucleic acid (HNA).

For example, the substitution with a benzyl group may be substitution with 5'-(*N*-benzylcarboxyamide)-2'-deoxyuridine (Bn-dU), but is not limited thereto, and the Bn-dU may be represented by Chemical Formula 1 below.

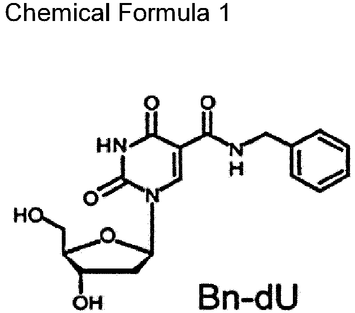

For example, the substitution with a naphthyl group may be substitution with 5-(*N*-1-naphthylmethylcarboxyamide)-2'-deoxyuridine (NapdU), but is not limited thereto.

For example, the substitution with a 2-naphthyl group may be substitution with 5-(*N*-(2-naphthylmethyl)carboxyamide)-2'-deoxyuridine (2NapdU), but is not limited thereto.

The NapdU may be represented by Chemical Formula 2 below, and the 2NapdU may be represented by Chemical Formula 3 below.

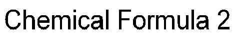

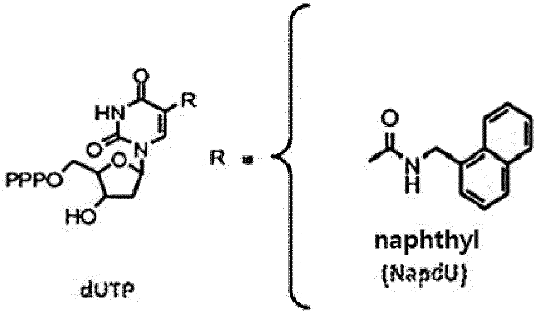

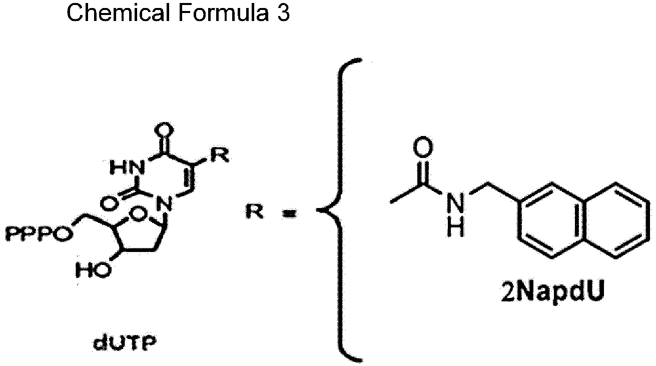

In the present invention, the aptamer including the above-described modification may include at least one nucleotide sequence selected from the group consisting of SEQ ID NOS: 5 to 49 or a nucleotide sequence having at least 90% homology therewith, without being limited thereto, and may be named "chemically optimized aptamer" or "TfR combi aptamer" below.

The aptamer of the present invention may have significantly high binding ability to the transferrin receptor protein, compared to non-modified cases, as the 5^{th} carbon of thymine (T) in the sequence is substituted with a particular functional group and/or the 2^{nd} carbon of at least one base selected from adenine (A), uracil (U), guanine (G), and cytosine (C) is substituted with a methoxy group.

In addition, the aptamer of the present invention may exhibit interspecific cross-reactivity.

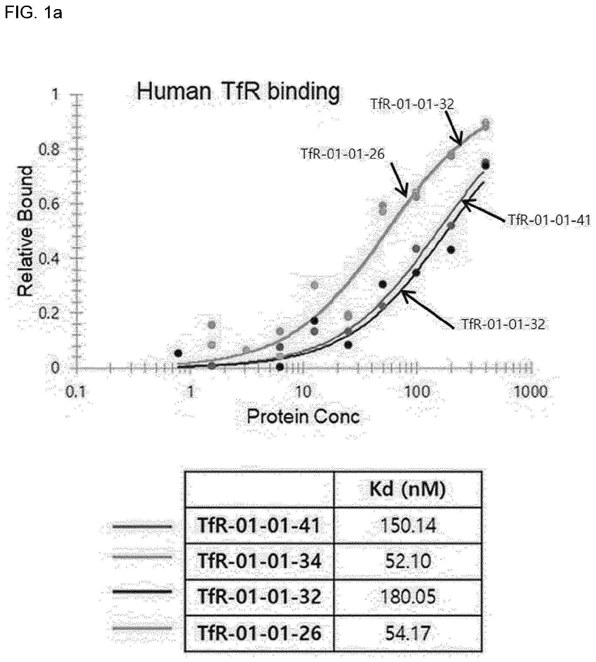

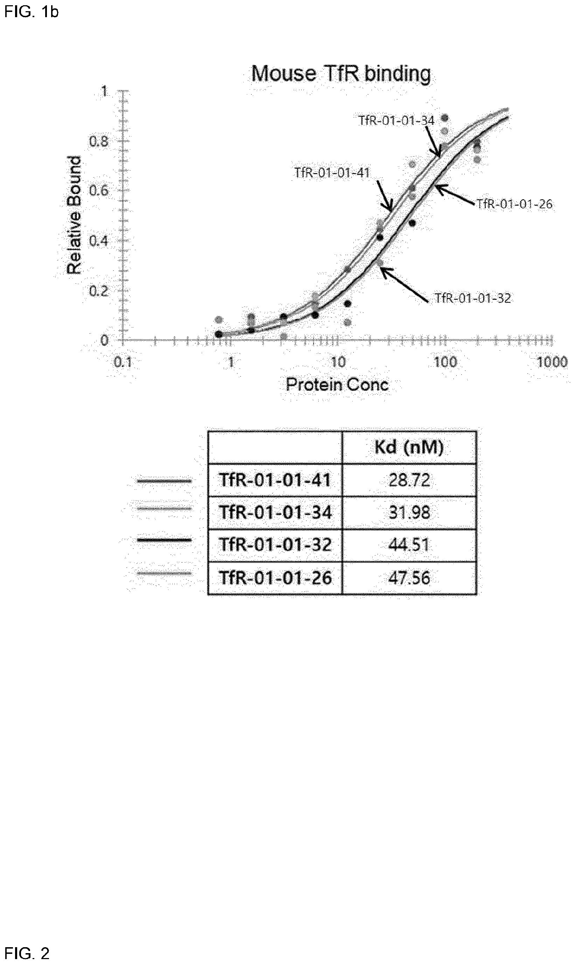

In an embodiment of the present invention, as a result of evaluating the ability of the TfR aptamer of the present invention to transferrin receptor proteins, excellent binding ability to human and mouse transferrin receptor proteins was confirmed (FIG. 1).

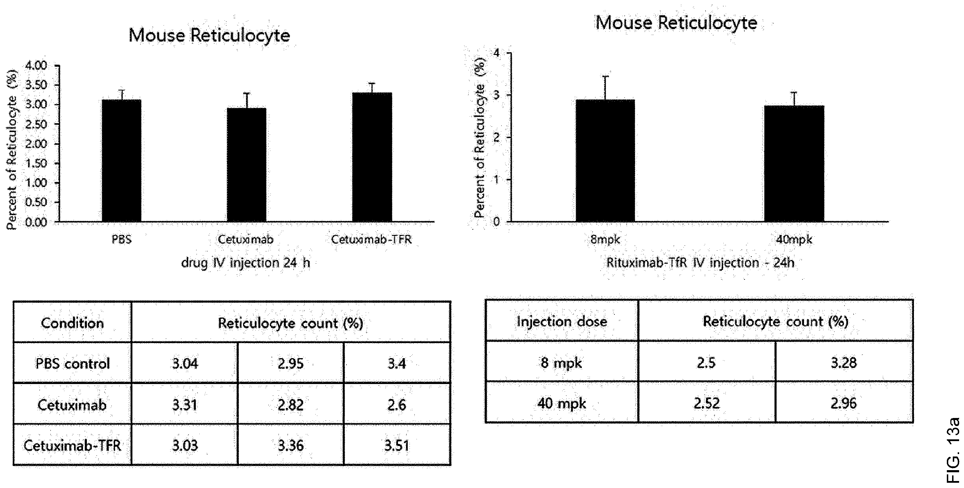

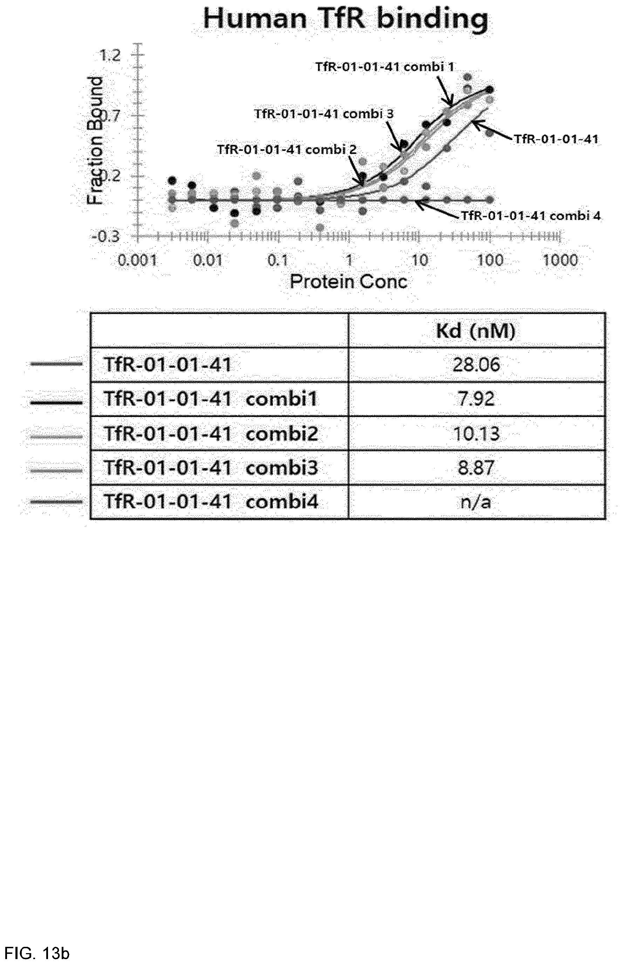

In another embodiment of the present invention, it was confirmed that chemically optimized TfR aptamers (SEQ ID NOS: 46 to 49) have improved transferrin receptor protein binding ability compared to the TfR aptamer (SEQ ID NO: 1) that is not chemically optimized (FIG. 13).

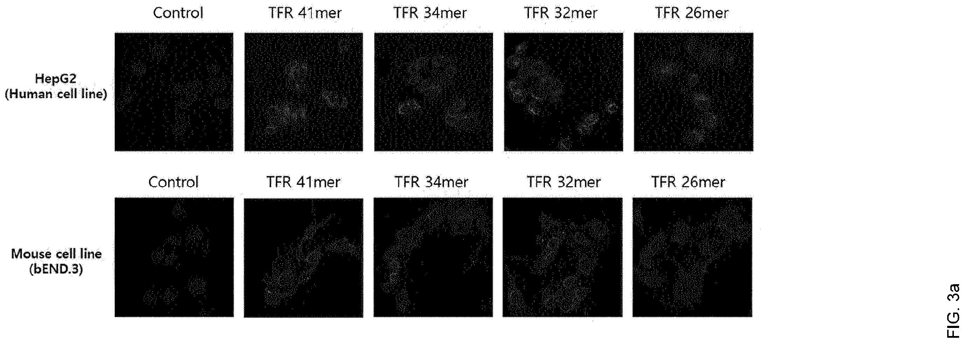

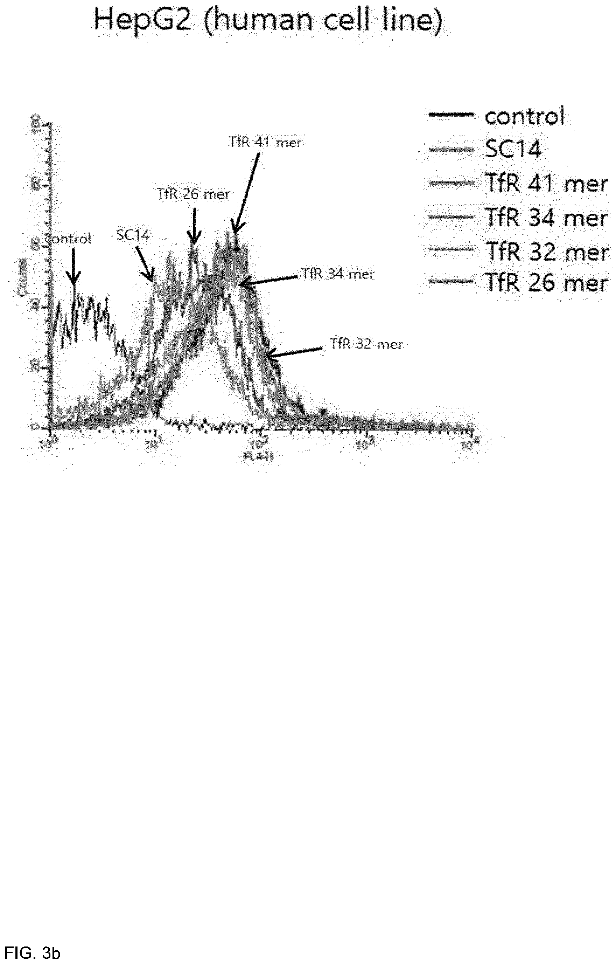

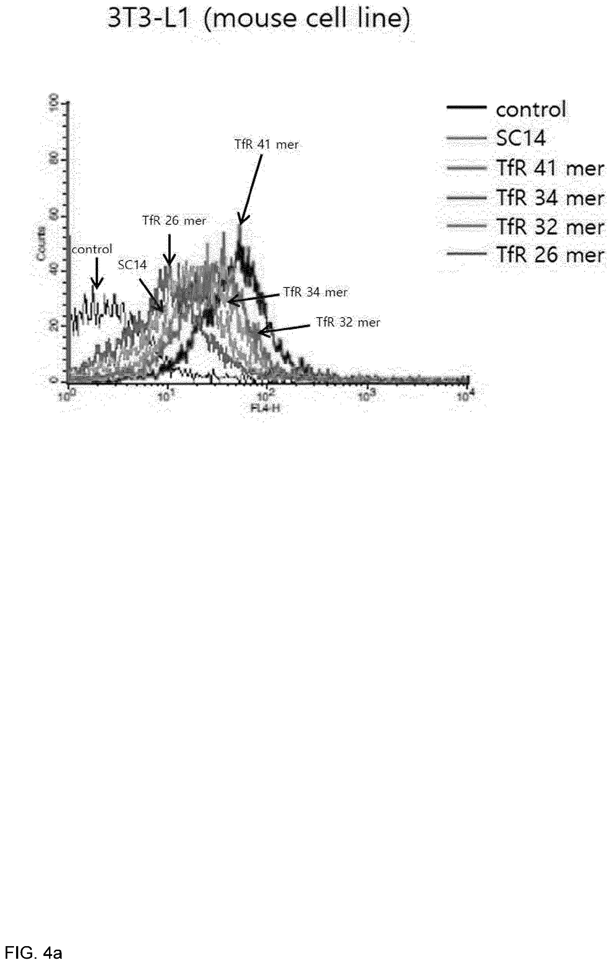

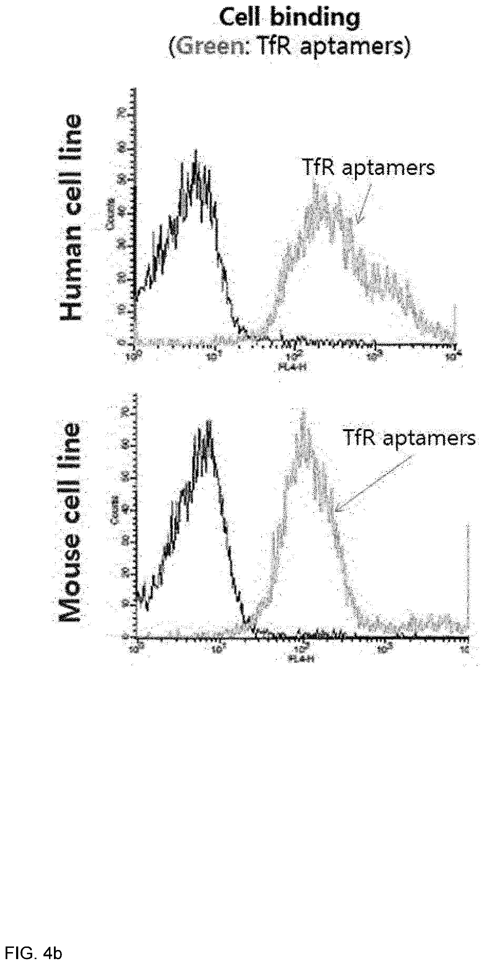

Also, in another embodiment of the present invention, it was confirmed that the TfR aptamers bind to both the human and mouse cell lines (FIGS. 3 and 4) and exhibit receptor-mediated endocytosis in both the human and mouse cell lines (FIG. 4).

The aptamer of the present invention may have excellent receptor-mediated endocytosis.

In an embodiment of the present invention, as a result of evaluating receptor-mediated endocytosis of TfR aptamers, receptor-mediated endocytosis was confirmed in both the human and mouse cells (FIGS. 2 to 4).

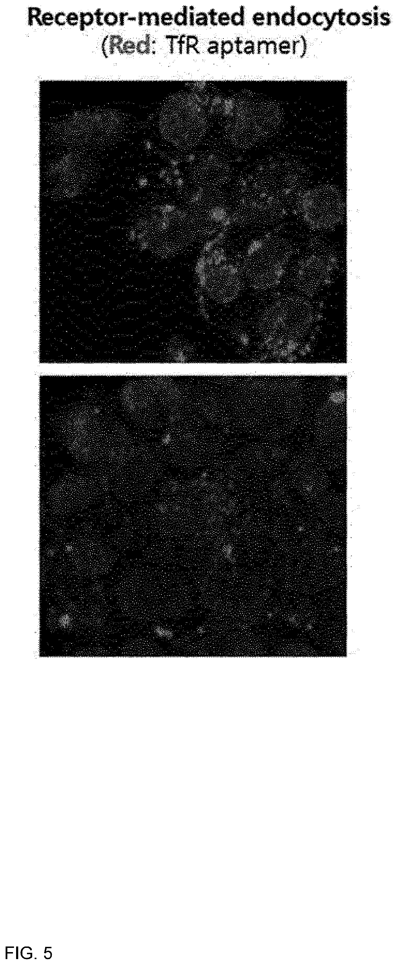

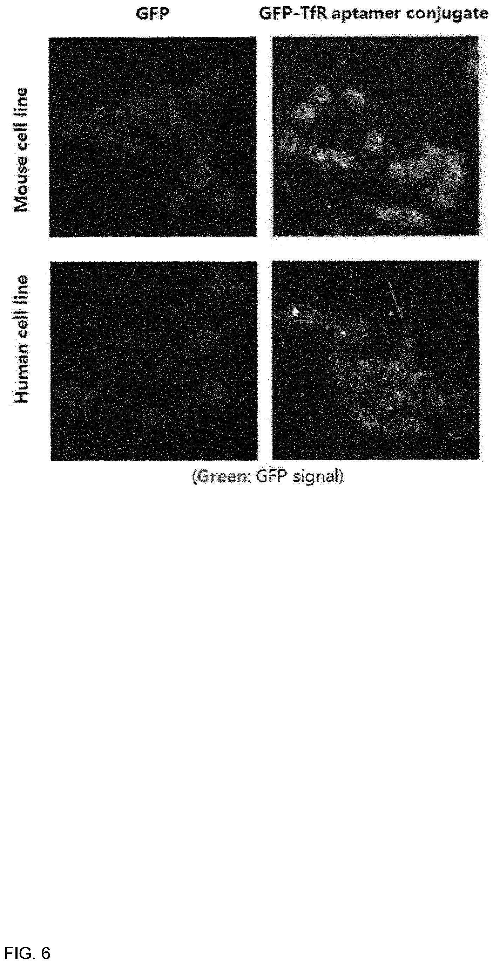

In another embodiment of the present invention, as a result of evaluating receptor-mediated endocytosis of the TfR aptamer conjugated with a protein or antibody, it was confirmed that a GFP-conjugated TfR aptamer exhibited receptor-mediated endocytosis both in the human and mouse cell lines (FIG. 5), and a Cetuximab- or Rituximab-conjugated TfR aptamer exhibited receptor-mediated endocytosis all in the human, monkey, and mouse cell lines (FIG. 6).

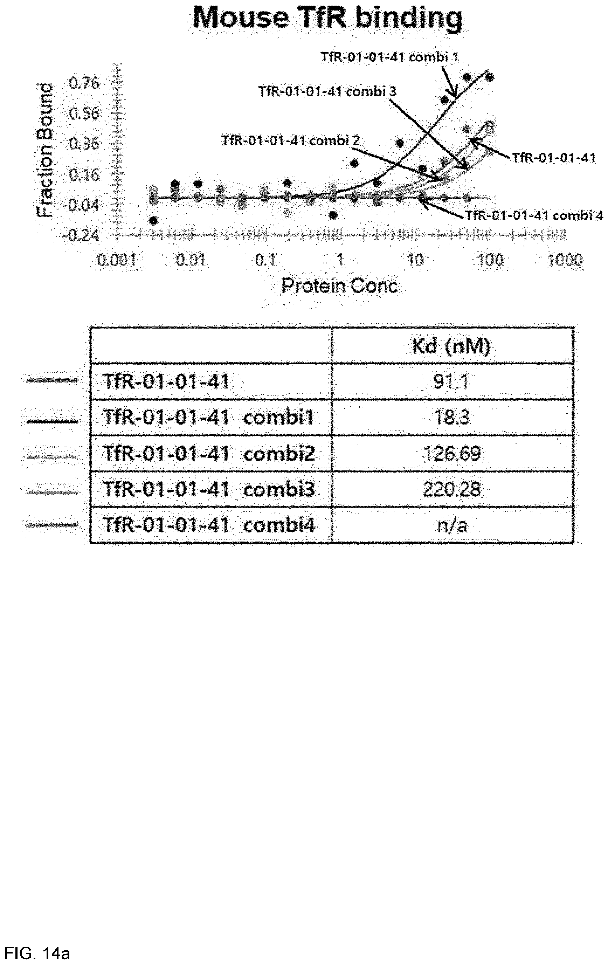

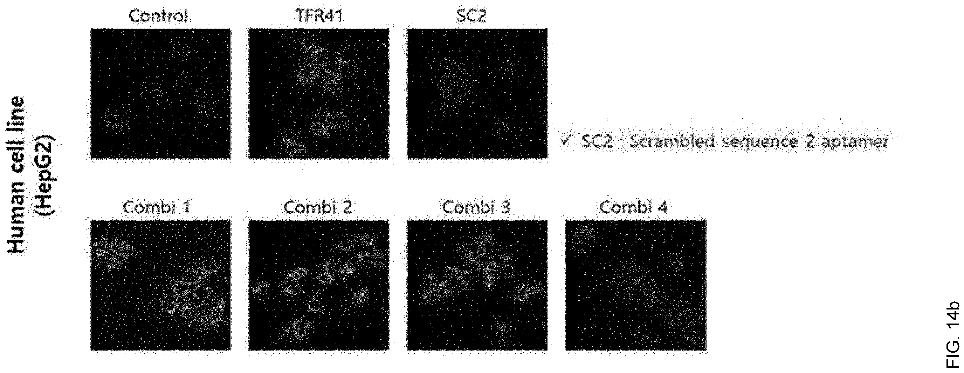

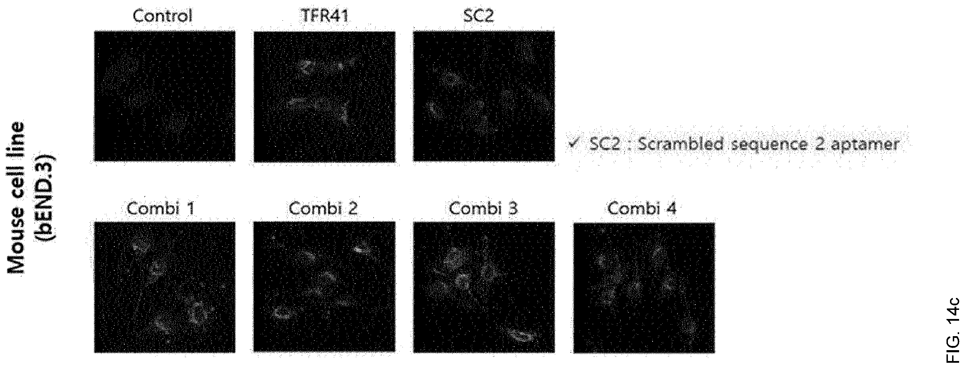

Also, in another embodiment of the present invention, it was confirmed that there was no difference in receptor-mediated endocytosis in the human, mouse, and monkey cell lines between the chemically optimized TfR aptamer and the TfR aptamer that is not chemically optimized (FIG. 14).

The aptamer of the present invention may penetrate the blood-brain barrier (BBB).

Also, the aptamer of the present invention may deliver a therapeutic agent such as an antibody, a protein, and a drug by penetrating the BBB.

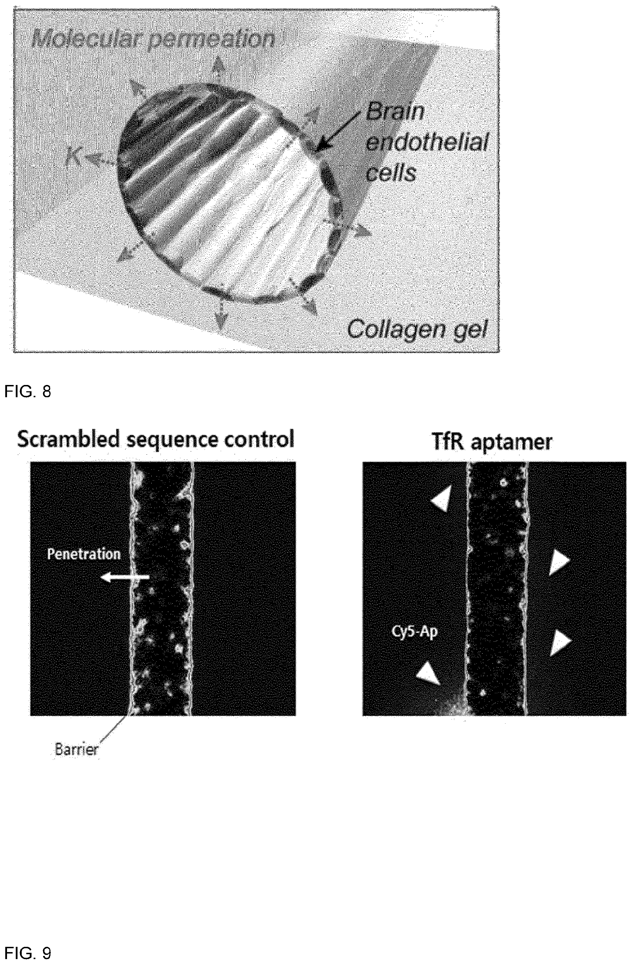

In an embodiment of the present invention, as a result of constructing an *in vitro* brain microvascular structure, and evaluating whether the TfR aptamer penetrates the structure, it was confirmed that the TfR aptamer penetrated the *in vitro* brain microvascular structure (FIG. 8).

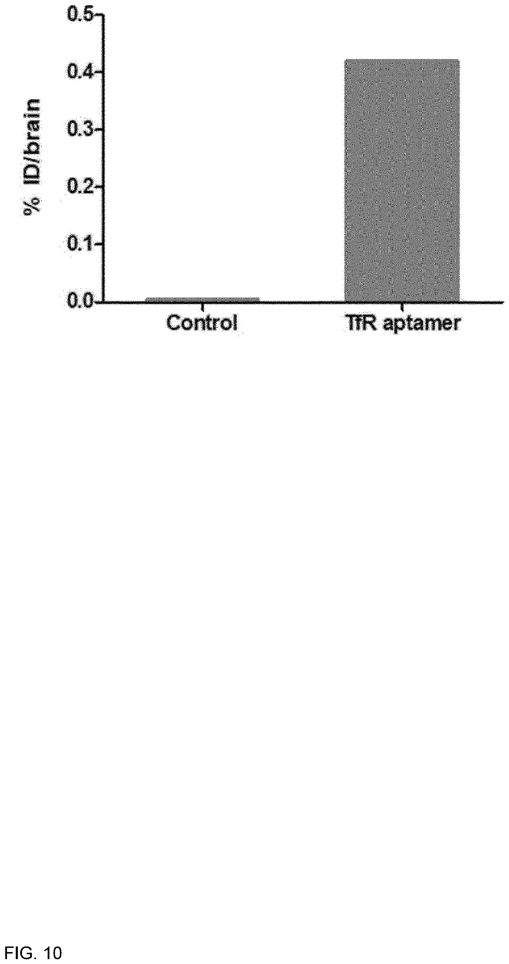

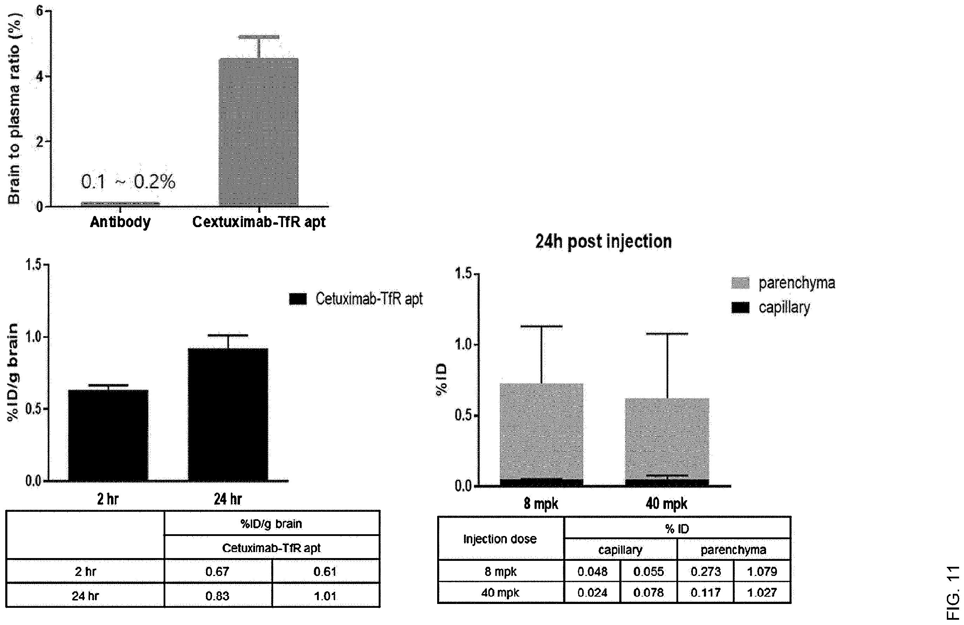

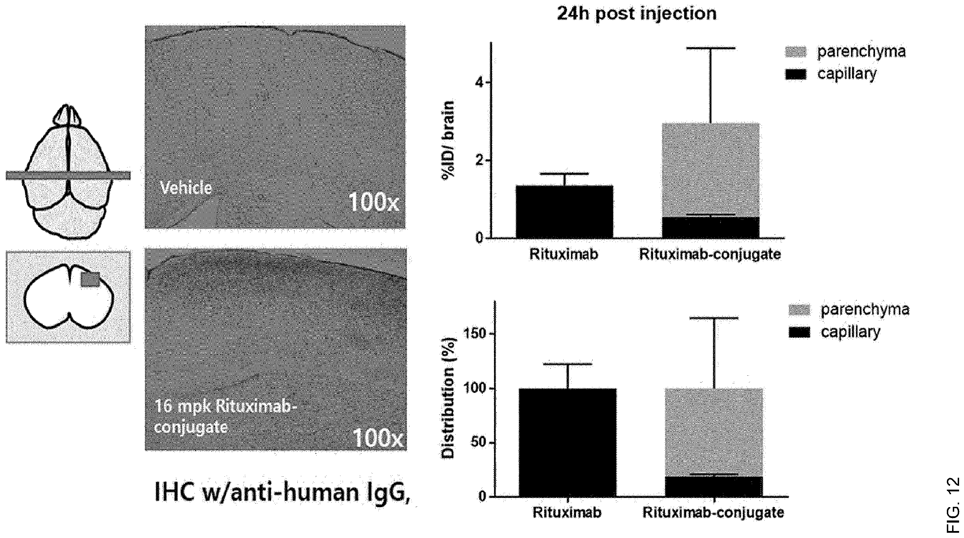

In another embodiment of the present invention, as a result of conducting an *in vivo* quantitative experiment on antibody distribution in brain in order to identify distribution of an antibody delivered to the brain by the TfR aptamer and an antibody-conjugated TfR aptamer, relative to the injected dose (ID), 0.42% of the TfR aptamer (FIG. 9) and 0.61 to 0.67% (2 hours after injection) and 0.83 to 1.01% (24 hours after injection) of the Cetuximab-conjugated aptamer (FIG. 10) were observed in the brain region, and it was confirmed that most of the antibody-conjugated TfR aptamers were present in parenchyma, and thus the TfR aptamer had significantly improved ability to penetrate the BBB in the case of being conjugated with the antibody. Also, it was confirmed that the Rituximab-conjugated TfR aptamer delivered an antibody to the entire brain, particularly, brain parenchyma (FIG. 11), and circulating reticulocytes was not reduced by the antibody-conjugated TfR aptamer (FIG. 12).

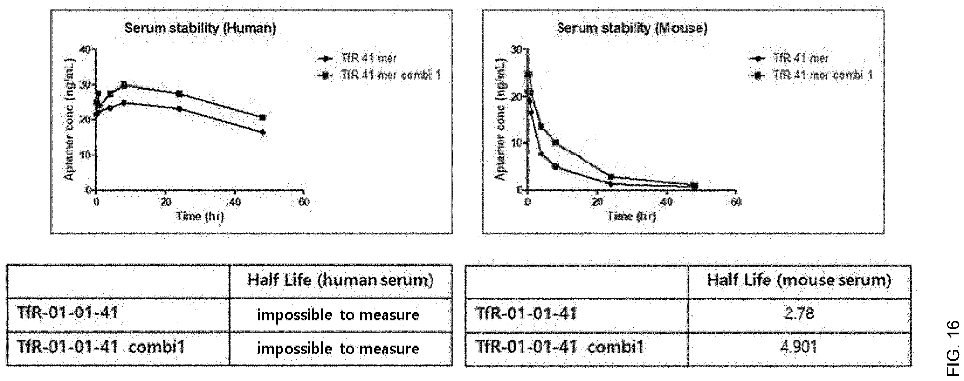

In addition, in another embodiment of the present invention, it was confirmed that the Rituximab-conjugated TfR combi 1 aptamer efficiently transported an antibody through cell membranes of human cells (FIG. 16).

Based thereon, it is confirmed that the TfR aptamer of the present invention may penetrate the BBB not only *in vitro* but also *in vivo,* and deliver a therapeutic agent such as an antibody, a protein, or a drug to brain parenchyma.

The aptamer of the present invention may have improved serum stability.

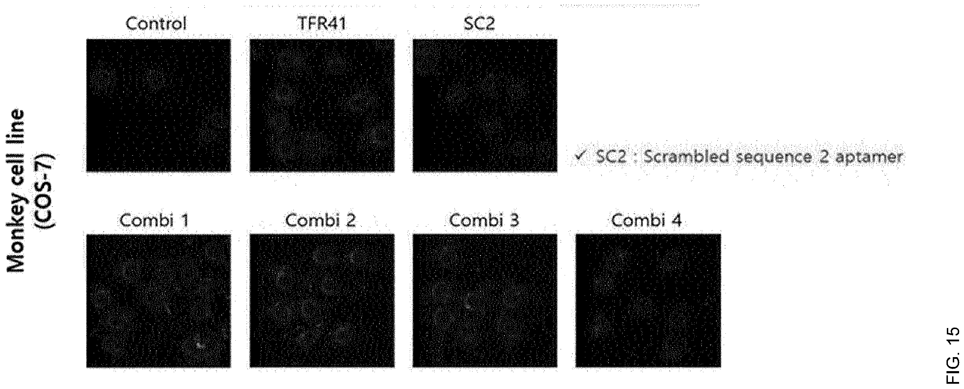

In an embodiment of the present invention, it was confirmed that the chemically optimized TfR aptamer had improved serum stability due to increased half-life in serum, compared with the TfR aptamer that is not chemically optimized (FIG. 15).

The aptamer may be conjugated with at least one selected from the group consisting of polyethylene glycol (PEG), hexaethylene glycol (HEG), inverted deoxythymidine (idT), biotin, a fluorescent substance, an amine linker, a thiol linker, cholesterol, and a fatty acid, without being limited thereto, at one or more of the 5'-terminus and the 3'-terminus of the nucleotide sequence thereof.

Another aspect of the present invention provides a pharmaceutical composition for preventing or treating a nervous system disease including the aptamer of the present invention as an active ingredient.

As used herein, the term "nervous system disease" refers to a disease caused by disorder occurring in a nervous system, *i.e.,* brain, spinal cord, and nerves, and may include several mental diseases. In view of the objects of the present invention, the nervous system disease may be a central nervous system disease.

As used herein, the term "central nervous system disease (CNS disease)" refers to a disease caused in brain and spinal cord.

The central nervous system disease may be, for example, brain cancer, brain infection, dementia, Parkinson's disease, Alzheimer's disease, Pick's disease, Huntington's disease, multiple sclerosis, epilepsy, stroke, cerebral apoplexy, ischemic brain disease, memory loss, traumatic central nervous system disease, spinal cord injury disease, behavioral disorder, developmental disability, mental retardation, Hunter's disease, amyotrophic lateral sclerosis (ALS), Down syndrome, and schizophrenia, without being limited thereto.

The aptamer of the present invention may be an aptamer additionally conjugated with at least one selected from the group consisting of a protein, an antibody, a nucleic acid, and a low-molecular weight compound.

The protein, antibody, nucleic acid, and low-molecular weight compound may be, for example, an anticancer drug, an antiviral agent, an antibiotic, or the like, without being limited thereto.

As used herein, the term "anticancer drug" includes prophylactic and therapeutic agents for cancer causing peripheral neuropathy as a side effect such as lung cancer (*e.g.,* non-small cell lung cancer, small cell lung cancer, or malignant mesothelioma), mesothelioma, pancreatic cancer (*e.g.,* pancreatic duct cancer or pancreatic endocrine tumor), pharyngeal cancer, laryngeal cancer, esophageal cancer, gastric cancer (*e.g.,* papillary adenocarcinoma, mucinous adenocarcinoma, or adenosquamous carcinoma), duodenal cancer, small intestine cancer, colorectal cancer (*e.g.,* colon cancer, rectal cancer, anal cancer, familial colorectal cancer, hereditary nonpolyposis colorectal cancer, or gastrointestinal stromal tumor), breast cancer (*e.g.,* invasive ductal cancer, non-invasive ductal cancer, or inflammatory breast cancer), ovarian cancer (*e.g.,* epithelial ovarian carcinoma, extra-testicular germ cell tumor, ovarian germ cell tumor, or ovarian low grade serious tumor), testis cancer, prostate cancer (*e.g.,* hormone-dependent prostate cancer or hormone-independent prostate cancer), thyroid cancer (*e.g.,* medullary thyroid carcinoma), kidney cancer (*e.g.,* renal cell carcinoma or transitional cell carcinoma of the renal pelvis and ureter), uterine cancer (*e.g.,* cervical cancer, cancer of uterine body, or uterine sarcoma), brain cancer or brain tumor (*e.g.,* medulloblastoma, glioma, pineal astrocytoma, pilocytic astrocytoma, diffuse astrocytoma, anaplastic astrocytoma, or pituitary adenoma), retinoblastoma, skin cancer (*e.g.,* basal cell carcinoma or malignant melanoma), sarcoma (*e.g.,* rhabdomyosarcoma, leiomyosarcoma, or soft tissue sarcoma), malignant bone tumor, bladder cancer, blood cancer (*e.g.,* multiple myeloma, leukemia, malignant lymphoma, Hodgkin's disease, or chronic myeloproliferative disease), or cancer of unknown primary. Examples of the anticancer drug may include Cetuximab (Erbitux), Rituximab (Rituxan), Atezolizumab (Tecentriq), Lenalidomide (Revlimid), Treosulfan (Treosulfan), Imatinib mesylate (Glivec), Dexamethasone (Dexamethasone Inj), Gemcitabine (Gemcitabine Inj), Carboplatin (Paraplatin), Cisplatin (Platinol, Platinol-AQ), Crizotinib (Xalkori), Cyclophosphamide (Cytoxan, Neosar), Docetaxel (Taxptere), Doxorubicin (Adriamycin), Erlotinib (Tarceva), Etoposide (Vepesid), 5-fluorouracil (5-FU), Irinotecan (Camptosar), liposome-capsulated doxorubicin (Doxil), Methotrexate (Folex, Mexate, Amethopterin), Paclitaxel (Taxol, Abraxane), Sorafenib (Nexavar), Sunitinib (Sutent), Topotecan (Hycamtin), Trabectedin (Yondelis), Vincristine (Oncovin, Vincasar PFS), and Vinbrastine (Velban), but are not limited thereto, and any anticancer drugs known in the art may be used without limitation. Specifically, the anticancer drug may include at least one selected from the group consisting of lenalidomide, Treosulfan and Imatinib mesylate, and Dexamethasone, without being limited thereto.

The pharmaceutical composition of the present invention has a use for "prevention" and/or "treatment" of a nervous system disease. For preventive use, the pharmaceutical composition of the present invention may be administered to an individual having or suspected to have a risk of developing a disease, disorder, or condition described herein, *i.e.,* individual having a risk of occurrence of a nervous system disease. For therapeutic use, the pharmaceutical composition of the present invention is administered to an individual such as a patient already suffering from the disease, disorder, or condition described herein in an amount sufficient to treat or at least partially cease symptoms of the disease, disorder, or condition. An effective amount for this use may vary according to severity and progression of the disease, disorder, or condition, previous history of treatment, health status and drug sensitivity of an individual, and judgement of doctors or veterinarians.

The pharmaceutical composition of the present invention may further include a suitable carrier, excipient, or diluent commonly used for preparation thereof. In this regard, the amount of the active ingredient contained in the composition may be, but is not limited to, 0.0001 wt% to 10 wt%, specifically 0.001 wt% to 1 wt%, based on a total weight of the composition.

The pharmaceutical composition may be formulated in any oral or parenteral formulation selected from the group consisting of tablets, pills, powders, granules, capsules, suspensions, formulations for internal use, emulsions, syrups, sterilized aqueous solutions, non-aqueous solvents, lyophilized preparations, and suppositories. For formulation of the composition, common diluents or excipients such as fillers, extenders, binders, humectants, disintegrants, and surfactants may be used. Solid formulations for oral administration may include tablets, pills, powders, granules, capsules, *etc.* and may be prepared by adding at least one excipient, *e.g.,* starch, calcium carbonate, sucrose or lactose, and gelatin, to at least one compound. Also, lubricants such as magnesium stearate and talc may be used in addition to simple excipients. Liquid formulations for oral administration may include suspensions, formulations for internal use, emulsions, syrups, *etc.,* and various kinds of excipients, such as humectants, sweeteners, fragrances, and preservatives, may be used in addition to simple diluents such as water and liquid paraffin. Formulations for parenteral administration may include sterile aqueous solutions, non-aqueous solvents, suspensions, emulsions, lyophilized preparations, and suppositories. For non-aqueous solvents and suspensions, propylene glycol, polyethylene glycol, a vegetable oil such as olive oil, and an injectable ester such as ethyl oleate may be used. Examples of bases for suppositories may include Witepsol, macrogol, Tween 61, cacao butter, laurin butter, glycerogelatin, *etc.*

The composition of the present invention may be administered to an individual in a pharmaceutically effective amount.

As used herein, the term "pharmaceutically effective amount" refers to an amount sufficient for treatment of diseases at a reasonable benefit/risk ratio applicable to medical treatment, and the level of an effective dose may be determined based on the factors including the type of the individual, severity of illness, age or gender of the individual, type of disease, drug activity, sensitivity to drug, administration time, administration route and excretion rate, duration of treatment, factors including drug(s) to be concurrently used in combination, and other factors well known in the medical field. The composition of the present invention may be administered alone as an individual therapeutic agent, in combination with other therapeutic agents, or sequentially or simultaneously with a conventional therapeutic agent, and may be administered once or multiple times. It is important to administer a minimum amount to obtain a maximum effect without side effects considering the factors described above, and the amount may be easily determined by one of ordinary skill in the art. An appropriate dose of the composition of the present invention varies the status of a patient, severity of disease, formulation of a drug, administration route, and duration of administration, and administration may be done once a day or divided several times during a day. The composition is not particularly limited as long as the composition is administered to an individual for prevention or treatment of a nervous system disease. Any administration method commonly used in the art may be used without limitation. For example, the composition may be administered by oral, rectal or intravenous, intramuscular, subcutaneous, intrauterine epidural, or intracerebrovascular injections.

By administering the pharmaceutical composition of the present invention to an individual having a nervous system disease or suspected to have a risk of developing the disease, occurrence of the nervous system disease may be prevented or the degree of occurrence may be reduced.

In the pharmaceutical composition of the present invention, the aptamer or the aptamer conjugated with at least one selected from the group consisting of a protein, an antibody, a nucleic acid, and a low-molecular weight compound, used as an active ingredient, may be included in an amount of 0.0001 wt% to 10 wt%, specifically 0.001 wt% to 1 wt%, without being limited thereto.

Another aspect of the present invention provides a drug carrier including the aptamer of the present invention.

The terms used herein are as described above.

The drug carrier may further include at least one selected from the group consisting of a protein, an antibody, a nucleic acid, and a low-molecular weight compound, without being limited thereto.

The protein, antibody, nucleic acid, and low-molecular weight compound may be, for example, an anticancer drug, an antiviral agent, an antibiotic, or the like as described above.

Another aspect of the present invention provides a method of preventing or treating a nervous system disease including administering the pharmaceutical composition or the drug carrier to an individual.

The terms used herein are as described above.

As used herein, the term "individual" refers to any animal having a nervous system disease or at the risk of developing the nervous system disease, and the pharmaceutical composition or the drug carrier of the present invention may effectively treat the individual suspected to have the nervous system disease by administering the pharmaceutical composition or the drug carrier to the individual.

As used herein, the term "administration" refers to introduction of the pharmaceutical composition or the drug carrier of the present invention into an individual suspected to have a nervous system disease by any suitable method, and an administration route may be any oral or parenteral route that enables delivery thereof to a target tissue.

The pharmaceutical composition of the present invention may be administered in a pharmaceutically effective amount and the pharmaceutically effective amount is as described above.

The pharmaceutical composition or the drug carrier of the present invention may be applied to any individual without limitation as long as the application is conducted for the purpose of preventing or treating nervous system diseases. For example, the individual may be any non-human animals such as monkey, dog, cat, rabbit, marmot, rat, mouse, cow, sheep, pig, and goat, birds, and fish, and the pharmaceutical composition or the drug carrier may be administered via parenteral, subcutaneous, intraperitoneal, intrapulmonary, and intranasal routes or may be administered, if required, by a suitable method including intralesional injection for topical treatment. A preferable dosage of the pharmaceutical composition or the drug carrier of the present invention may vary according to the status and body weight of the individual, severity of disease, formulation of drug, and administration route and duration but may be appropriately selected by one or ordinary skill in the art. For example, the pharmaceutical composition or the drug carrier may be administered by an oral, rectal or intravenous, intramuscular, subcutaneous, or intrauterine route, or intracerebrovascular injection, without being limited thereto.

An appropriate daily dose of the pharmaceutical composition or the drug carrier of the present invention may be determined by a doctor within the scope of sound medical judgment. In general, the pharmaceutical composition or the drug carrier may be administered in an amount of 0.001 mg/kg to 1000 mg/kg, specifically 0.05 mg/kg to 200 mg/kg, more specifically 0.1 mg/kg to 100 mg/kg, once a day or divided several times a day.

### Mode for Invention

Hereinafter, the present invention will be described in more detail with reference to the following examples. However, the following examples are merely presented to exemplify the present invention, and the scope of the present invention is not limited thereto.

### [Examples]

### Example 1. Construction of Transferrin Receptor (TfR) Aptamer and Identification of Transferrin Receptor Protein Binding Ability and Cell Binding Ability Thereof

### 1-1. Construction of TfR Aptamer

TfR aptamers were constructed by developing aptamers binding to human transferrin receptor proteins through systematic evolution of ligands by exponential enrichment (SELE), which is a common process of developing aptamers, and chemically synthesizing selected sequences. Thus, TfR aptamers having SEQ ID NOS: 1 to 4 were constructed (Table 1 below). The constructed aptamers had lengths of 41mer, 34mer, 32mer, and 26mer, respectively, and each aptamer had a size less than 15 kDa.

**Table 1**

| SEQ ID NO: | Name | Sequence (5'→3') |
|---|---|---|
| 1 | TfR-01-01-41 | GAABBCAAAAGBGBCGGBGABBBGCCBCGBCGC BCCAABGB |
| 2 | TfR-01-01-34 | GAABBCAAAAGBGBCGGBGABBBGCCBCGBCGC B |
| 3 | TfR-01-01-32 | AGBGBCGGBGABBBGCCBCGBCGCBCCAABGB |
| 4 | TfR-01-01-26 | AAGBGBCGGBGABBBGCCBCGBCGCB |

The B represents a base substituted with 5'-(N-benzylcarboxyamide)-2'-deoxyuridine (Bn-dU).

### Example 2. Identification of Transferrin Receptor Protein Binding Ability and Cell Binding Ability of TfR Aptamer

### 2-1. Transferrin Receptor Protein Binding Ability of TfR Aptamer

In order to identify transferrin receptor protein binding ability of the constructed TfR aptamers, each of the aptamers was added to a 1X SB18 buffer (containing 40 mM hydroxyethyl piperazine ethane sulfonic acid (HEPES), 105 mM NaCl, 5 mM MgCl₂, 5 mM KCI, and 0.05% [v/v] tween-20) and reacted at 95°C, 70°C, 48°C, and 37°C, respectively, for 5 minutes. Human or mouse transferrin receptor proteins were diluted in series with 7 points from 100 nM at a dilution ratio of 4.64 times using the 1X SB18 buffer, and then 30 µL of each of the aptamers (SEQ ID NOS: 1 to 4) was added thereto, followed by reaction at 37°C for 30 minutes. 5.5 µL of Dynabeads^{®} TALON^{™} were added to the aptamer-protein conjugates, followed by reaction at room temperature for 5 minutes. The aptamer-protein conjugates were added to a Durapore^{®} filter plate previously soaked with 30 µL of the 1X SB18 buffer and a vacuum was applied thereto. Subsequently, 100 µL of the 1X SB18 buffer was added thereto, and a vacuum was applied thereto for rinsing. Thereafter, a 2 mM NaOH solution was added thereto, followed by reaction at room temperature for 5 minutes and elution. The elute was neutralized with an 8 mM HCl solution, and the aptamer-protein conjugates were amplified by qPCR. After mixing 0.2 µM forward primer (SEQ ID NO: 50) and reverse primer (SEQ ID NO: 51), 0.2 µM dNTP (dATP, dGTP, dCTP, and dTTP), 5 mM MgCl₂, 0.025 U/µL KOD XL DNA polymerase, and DNA-protein complexes to a total volume of 20 µL, the qPCR was performed once under the conditions of 96°C for 15 seconds, 55°C for 10 seconds, and 70°C for 30 minutes and 40 times under the conditions of 96°C for 15 seconds, 55°C for 10 seconds, and 70°C for 1 minute. After normalizing the Ct value to a standard, K_{d} was calculated using SigmaPlot.

As a result, it was confirmed that aptamers having short sequences had excellent binding ability to the human and mouse transferrin receptor proteins as shown in FIG. 1.

### 2-2. Cell Binding Ability of TfR Aptamer

In order to identify cell binding ability of the constructed aptamers, a cover glass was sterilized and placed under the bottom surface of a 6-well plate, and then human HepG2 cells or mouse bEND.3 cells were seeded at a density of 1x10⁶ cells/well and incubated. When about 70% of the bottom surface was filled with the cells, the culture medium was removed and the cells were rinsed with 1X PBS. The cells were treated with 2 mL of a KRPH buffer. 25 nM of aptamers (SEQ ID NOS: 1 to 4) prepared by heating at 95°C for 5 minutes and then cooling at room temperature for 15 minutes were applied to the cells and incubated at 37°C under 5% CO₂ conditions. After 4 hours, a process of adding 1X PBS, allowing to stand at room temperature for 5 minutes, and removing the 1X PBS was repeated three times. The cells were immobilized with 2 mL of 4% paraformaldehyde at 4°C for 15 minutes. After removing the 4% paraformaldehyde, a process of adding 1X PBS thereto, allowing to stand at room temperature for 5 minutes, and removing the 1X PBS was repeated three times, and then the cells were stained using a mounting solution and sealed. The cells were stored in a light-blocking box in a refrigerated state and measured next day using a confocal microscope (LSM800).

As a result, as shown in FIG. 2, it was confirmed that aptamers having short sequences exhibited receptor-mediated endocytosis in humans and mice.

### Example 3. In Vitro Cell Binding Ability and Receptor-mediated Endocytosis of TfR Aptamer

### 3-1. Cell Binding Ability and Receptor-mediated Endocytosis of TfR Aptamer

In order to identify cell binding ability of the TfR aptamers, human HepG2 cells or mouse_3T3-L1 cells were applied onto a 100 mm² dish at a density of 2×10⁶ cells/mL and incubated for 24 hours at 37°C under 5% CO₂ conditions. The cultured cells were rinsed with 1X PBS (phosphate-buffered saline). After removing the 1X PBS, a growth medium was added thereto, and the cells were scrapped using a scraper and transferred to a FACS tube, followed by centrifugation at 2000 rpm for 3 minutes at 4°C to remove a supernatant. A Cy5-TfR aptamer prepared by labeling the 5'-terminus of the TfR aptamer (TfR-01-01-41, SEQ ID NO: 1) with Cy5 was heated at 95°C for 5 minutes and cooled at room temperature for 15 minutes, and then the Cy5-TfR aptamer was diluted with a KRPH buffer (containing 20 mM HEPES, 5 mM KH₂PO₄, 1 mM MgSO₄, 1 mM CaCl₂, 136 mM NaCl, and 4.7 mM KCI, pH 7.4) to a desired concentration and applied to the cells at 4°C under light-blocking conditions. After 30 minutes, a process of applying 2 mL of 1X PBS, pipetting, and centrifuging to remove a supernatant was repeated three times. After removing the supernatant, 1X PBS was added thereto, and the cells were transferred to a new strainer cap FACS tube and measured using a flow cytometry device (BD FACS Calibur).

Subsequently, receptor-mediated endocytosis was identified using the human HepG2 cells and the mouse bEND.3 cells in the same manner as in Example 2-2.

As a result, it was confirmed that the TfR aptamers bound to both the human and mouse cell lines (FIGS. 3 and 4) and exhibited receptor-mediated endocytosis in the human and mouse cell lines (FIG. 4).

### 3-2. Receptor-mediated Endocytosis of Protein- or Antibody-conjugated TfR Aptamer

Human HepG2 cells, mouse bEND.3 cells, or monkey Cos7 cells were incubated in the same manner as in Example 3-1 and rinsed with 1X PBS. While incubating the cells, a green fluorescence protein (GFP)-conjugated TfR aptamer, a TfR aptamer conjugated with Cetuximab, which is an epidermal growth factor receptor inhibitor as a monoclonal antibody, and a TfR aptamer conjugated with Rituximab, which is a monoclonal antibody against antigen CD20 found on the surface immune system B cells were diluted with a 1X SB18 buffer (containing 40 mM HEPES, 105 mM NaCl, 5 mM KCI, 5 mM MgCl₂, 0.05% [v/v] Tween-20, and 0.1 mg mL-1 bovine serum albumin, pH 8.0) to desired stock concentrations and allowed to stand at room temperature for 1 hour. The TfR aptamers (TfR-01-01-41, SEQ ID NO: 1) conjugated with the protein or antibodies were mixed with a KRPH buffer to a concentration of 50 nM, and the cells were treated with 2 mL of the diluted TfR aptamers and incubated for 4 hours at 37°C under 5% CO₂ conditions. After 4 hours, the KRPH buffer was removed, and a process of adding 2 mL of 1X PBS, allowing to stand for 5 minutes, and removing the 1X PBS was repeated three times. Subsequently, the cells were immobilized with 2 mL of 4% paraformaldehyde at 4°C for 15 minutes. After removing the 4% paraformaldehyde, a process of adding 2 mL of 1X PBS, allowing to stand at room temperature for 5 minutes, and removing the 1X PBS was repeated three times. Then, a permeabilization buffer (0.2% Triton X-100 in 1X PBS) was added thereto and the cells were left to stand at room temperature for 15 minutes. After removing the permeabilization buffer, a process of adding 2 mL of 1X PBS, allowing to stand at room temperature for 5 minutes, and removing the 1X PBS was repeated three times.

Subsequently, after a blocking solution (3% bovine serum albumin (BSA) + 1X PBS) was added, the cells were incubated for 20 minutes at 37°C under 5% CO₂ conditions. After adding 2 mL of 1X PBS thereto, the cells were left to stand at room temperature for 5 minutes, and then the 1X PBS was removed. 2 mL of a blocking solution containing human secondary antibody-fluorescein isothiocyanate (FITC) (52230-0425) at a concentration of 5 µg/mL was added to a 6-well plate, followed by incubation for 1 hour and 30 minutes at 37°C under 5% CO₂ conditions. After removing the secondary antibody, the cells were rinsed by repeating a process of adding 2 mL of 1X PBS, allowing to stand at room temperature for 5 minutes, and removing the 1X PBS three times. The cells were stained using a mounting solution and sealed, and then stored in a light-blocking box in a refrigerated state and measured next day using a confocal microscope (LSM800).

As a result, it was confirmed that the GFP-conjugated TfR aptamer exhibited receptor-mediated endocytosis in both the human and mouse cell lines as shown in FIG. 5, and the Cetuximab- or Rituximab-conjugated TfR aptamer exhibited receptor-mediated endocytosis in all of the human, monkey, and mouse cell lines as shown in FIG. 6.

### Example 4. Identification of Effect of TfR Aptamer on In Vitro Blood-brain Barrier (BBB) Penetration

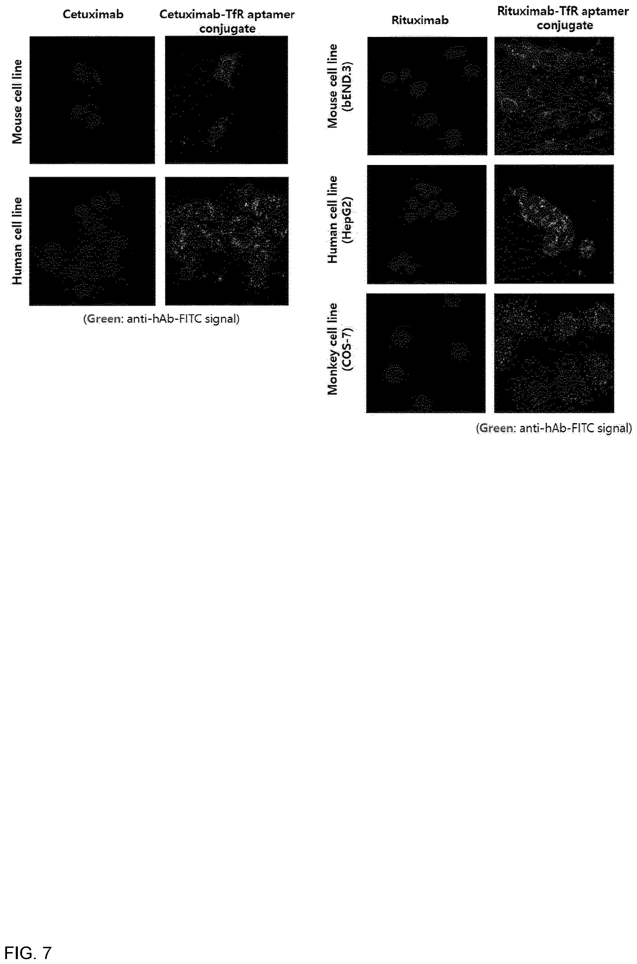

Effects of the TfR aptamers on penetrating blood-brain barrier (BBB) were identified *in vitro* by using a brain microvascular structure (brain microvasculature model, J. A. Kim et al., Biomicrofluidics, 2015) (FIG. 7).

Specifically, in order to construct a brain microvascular structure, the inner surface of a square opening in a 3D-printed frame and a square cover glass were treated sequentially with 1% [v/v] polyethyleneimine (PEI) for 30 minutes and 0.1% [v/v] glutaraldehyde for 30 minutes. Then, the surface coated with glutaraldehyde was completely rinsed twice with distilled water. After microneedles were inserted through cylindrical microholes in the 3D-printed frame and the 3D-printed frame was assembled, a previously mixed collagen solution was slowly applied thereto until the square opening where the microneedles and the glutaraldehyde-coated cover glass were located was filled. Then, the top was slowly covered to press and confine the gel. Three layers were fixed with sterilized stainless steel screws to prevent leakage of the gel. Collagen in the assembled device was allowed for gelation in a CO₂ incubator at 37°C for 30 minutes. After the gelation, the microneedles were removed to form hollow tubular microvessels in the gelled collagen. To promote adhesion of bEnd.3 cells, inner surfaces of the collagen microchannels were coated with 20 µg/mL of fibronectin for 30 minutes. The bEnd.3 cells were loaded into fibronectin-coated collagen microchannels, and then the device was placed in the incubator for 10 minutes. To increase areas of initial cell adhesion, the device was quickly flipped over and incubated for another 10 minutes. During culturing periods, a pair of extended reservoirs, which were separately fabricated, was connected to two adaptors in the 3D-printed frame. About 1.5 mL of a culture medium was filled in each reservoir and the culture medium was replaced every day during culture periods of up to 21 days.

In order to measure endothelial permeability via microvessels in the brain microvascular structure, the Cy5-TfR aptamer (TfR-01-01-41, SEQ ID NO: 1) was used. Immediately after filling the collagen microchannels with a Cy5-TfR aptamer solution, the flow was stopped for temporary interfacial diffusion. Fluorescent images of molecular transport were obtained for initial 5 minutes using a Zeiss LSM700 laser scanning confocal microscope. Then, the image was color-mapped and an average fluorescent intensity of the entire microchannels was analyzed with customized MATLAB (MathWorks) code.

As a result, as shown in FIG. 8, it was confirmed that the TfR aptamer penetrated the *in vitro* brain microvascular structure.

### Example 5. Identification of Antibody Delivery by TfR Aptamer and Antibody-conjugated TfR Aptamer

In order to identify distribution of an antibody delivered to the brain by the TfR aptamer and the antibody-conjugated TfR aptamer, an *in vivo* quantitative experiment on antibody distribution in the brain was conducted.

Specifically, Balb/c mice were administered with the TfR aptamer (TfR-01-01-41, SEQ ID NO: 1) and the TfR aptamer (TfR-01-01-41, SEQ ID NO: 1) conjugated with Cetuximab or Rituximab respectively via tail vein. After 2 or 24 hours, the mice were sacrificed to obtain plasma and brains. A physiological buffer was added to the brain tissue in a ratio of 3:1 (v/w), followed by homogenization on ice. An aliquot of the homogenized tissue was left and a 26% [v/v] dextran solution was added to the remaining tissue in an equal volume (to a final dextran concentration of 13%), followed by homogenization on ice again. The homogenized tissue was inoculated onto two microtubes (1.5 mL) and the 26% [v/v] dextran solution was added thereto in an equal volume. The mixture was centrifuged under the conditions of 5,400×*g* and 4°C for 15 minutes to separate a supernatant (brain parenchyma) from pellets (brain capillary). The homogenized tissue, brain capillary, and brain parenchyma were rinsed with 1X PBS and centrifuged at 14,000 rpm at 4°C for 10 minutes, and this process was repeated three times. After weighing pellets of each sample, the sample was dissolved in 1X RIPA buffer at a weight ratio of 2.5:1 v/w. After incubating for about 1 hour at 4°C, the resultant was centrifuged at 14,000 rpm at 4°C for 10 minutes, and only the supernatant was collected and transferred to a new tube. Then, concentrations of proteins contained in tissue lysates were measured.

As a result, relative to the injected dose (ID), 0.42% of the TfR aptamer (FIG. 9) and 0.61% to 0.67% (2 hours after injection) and 0.83% to 1.01% (24 hours after injection) of the Cetuximab-conjugated aptamer (FIG. 10), were observed in the brain region, and it was confirmed that most of the antibody-conjugated TfR aptamer was present in the parenchyma indicating that the BBB penetration was significantly improved when the TfR aptamer was conjugated with the antibody. In addition, it was confirmed that the Rituximab-conjugated TfR aptamer also delivered the antibody through the whole brain, particularly, to the brain parenchyma (FIG. 11).

### Example 6. Identification of Effect of Antibody-conjugated TfR Aptamer on In Vivo BBB Penetration and Circulating Reticulocytes

Effects of the antibody-conjugated TfR aptamers on *in vivo* BBB penetration and circulating reticulocytes were identified.

Specifically, Cetuximab, Rituximab, or Cetuximab- or Rituximab-conjugated TfR aptamer (TfR-01-01-41, SEQ ID NO: 1) was administered to Balb/c mice at a dose of 8 mpk (mg/kg) and 40 mpk via tail vein. Whole blood was collected and added to an EDTA tube and placed on ice. 1 mL of a BD-Retic-Count^{™} (Ca. 349204) reagent and 5 µL of the whole blood were mixed and incubated in a light-blocking state at room temperature for 30 minutes. The cells were transferred to a Strainer cap FACS tube and the number of reticulocytes in blood was analyzed using a flow cytometry device.

As a result, as shown in FIG. 12, it was confirmed that there was no reduction in circulating reticulocytes in the mice by the antibody-conjugated TfR aptamer.

### Example 7. Chemical Optimization of TfR Aptamer

In order to chemically optimize the aptamer (SEQ ID NO: 1) prepared in Example 1 above, bases in the nucleotide sequence of each aptamer was modified by methoxylation and the resultants are shown in Tables 2 to 5 below. In Tables 2 to 5 below, the B represents a base substituted with 5'-(*N*-benzylcarboxyamide)-2'-deoxyuridine (Bn-dU), and the N_{Me} (wherein N is one of A, U, G and C) represents a base in which an -OH group is substituted with a methoxy group of the 2^{nd} carbon of the nucleotide.

**Table 2**

| SEQ ID NO: | Name | Sequence (5'→3') |
|---|---|---|
| 5 | A_{Me} 1 | GA_{Me}ABBCAAAAGBGBCGGBGABBBGCCBCGBCG CBCCAABGB |
| 6 | A_{Me} 2 | GAAMeBBCAAAAGBGBCGGBGABBBGCCBCGBCG CBCCAABGB |
| 7 | A_{Me} 3 | GAABBCAMeAAAGBGBCGGBGABBBGCCBCGBCG CBCCAABGB |
| 8 | A_{Me} 4 | GAABBCAAMeAAGBGBCGGBGABBBGCCBCGBCG CBCCAABGB |
| 9 | A_{Me} 5 | GAABBCAAAMeAGBGBCGGBGABBBGCCBCGBCG CBCCAABGB |
| 10 | A_{Me} 6 | GAABBCAAAAMeGBGBCGGBGABBBGCCBCGBCG CBCCAABGB |
| 11 | A_{Me} 7 | GAABBCAAAAGBGBCGGBGA_{Me}BBBGCCBCGBCG CBCCAABGB |
| 12 | A_{Me} 8 | GAABBCAAAAGBGBCGGBGABBBGCCBCGBCGC BCCAMeABGB |
| 13 | A_{Me} 9 | GAABBCAAAAGBGBCGGBGABBBGCCBCGBCGC BCCAAMeBGB |

**Table 3**

| SEQ ID NO: | Name | Sequence (5'→3') |
|---|---|---|
| 14 | U_{Me} 1 | GAAU_{Me}BCAAAAGBGBCGGBGABBBGCCBCGBC GCBCCAABGB |
| 15 | U_{Me} 2 | GAABU_{Me}CAAAAGBGBCGGBGABBBGCCBCGBC GCBCCAABGB |
| 16 | U_{Me} 3 | GAABBCAAAAGU_{Me}GBCGGBGABBBGCCBCGBC GCBCCAABGB |
| 17 | U_{Me} 4 | GAABBCAAAAGBGUMeCGGBGABBBGCCBCGBC GCBCCAABGB |
| 18 | U_{Me} 5 | GAABBCAAAAGBGBCGGU_{Me}GABBBGCCBCGBC GCBCCAABGB |
| 19 | U_{Me} 6 | GAABBCAAAAGBGBCGGBGAU_{Me}BBGCCBCGBC GCBCCAABGB |
| 20 | U_{Me} 7 | GAABBCAAAAGBGBCGGBGABU_{Me}BGCCBCGBC GCBCCAABGB |
| 21 | U_{Me} 8 | GAABBCAAAAGBGBCGGBGABBU_{Me}GCCBCGBC GCBCCAABGB |
| 22 | U_{Me} 9 | GAABBCAAAAGBGBCGGBGABBBGCCU_{Me}CGBC GCBCCAABGB |
| 23 | U_{Me} 10 | GAABBCAAAAGBGBCGGBGABBBGCCBCGU_{Me}C GCBCCAABGB |
| 24 | U_{Me} 11 | GAABBCAAAAGBGBCGGBGABBBGCCBCGBCG CU_{Me}CCAABGB |
| 25 | U_{Me} 12 | GAABBCAAAAGBGBCGGBGABBBGCCBCGBCG CBCCAAU_{Me}GB |
| 26 | U_{Me} 13 | GAABBCAAAAGBGBCGGBGABBBGCCBCGBCG CBCCAABGU_{Me} |

**Table 4**

| SEQ ID NO: | Name | Sequence (5'→3') |
|---|---|---|
| 27 | G_{Me} 1 | GMeAABBCAAAAGBGBCGGBGABBBGCCBCGBC GCBCCAABGB |
| 28 | G_{Me} 2 | GAABBCAAAAG_{Me}BGBCGGBGABBBGCCBCGBC GCBCCAABGB |
| 29 | G_{Me} 3 | GAABBCAAAAGBG_{Me}BCGGBGABBBGCCBCGBC GCBCCAABGB |
| 30 | G_{Me} 4 | GAABBCAAAAGBGBCG_{Me}GBGABBBGCCBCGBC GCBCCAABGB |
| 31 | G_{Me} 5 | GAABBCAAAAGBGBCGG_{Me}BGABBBGCCBCGBC GCBCCAABGB |
| 32 | G_{Me} 6 | GAABBCAAAAGBGBCGGBG_{Me}ABBBGCCBCGBC GCBCCAABGB |
| 33 | G_{Me} 7 | GAABBCAAAAGBGBCGGBGABBBG_{Me}CCBCGBC GCBCCAABGB |
| 34 | G_{Me} 8 | GAABBCAAAAGBGBCGGBGABBBGCCBCGMeBC GCBCCAABGB |
| 35 | G_{Me} 9 | GAABBCAAAAGBGBCGGBGABBBGCCBCGBCGM eCBCCAABGB |
| 36 | G_{Me} 10 | GAABBCAAAAGBGBCGGBGABBBGCCBCGBCG CBCCAABGMeB |

**Table 5**

| SEQ ID NO: | Name | Sequence (5'→3') |
|---|---|---|
| 37 | C_{Me} 1 | GAABBC_{Me}AAAAGBGBCGGBGABBBGCCBCGBC GCBCCAABGB |
| 38 | C_{Me} 2 | GAABBCAAAAGBGBC_{Me}GGBGABBBGCCBCGBC GCBCCAABGB |
| 39 | C_{Me} 3 | GAABBCAAAAGBGBCGGBGABBBGC_{Me}CBCGBC GCBCCAABGB |
| 40 | C_{Me} 4 | GAABBCAAAAGBGBCGGBGABBBGCC_{Me}BCGBC GCBCCAABGB |
| 41 | C_{Me} 5 | GAABBCAAAAGBGBCGGBGABBBGCCBCMeGBC GCBCCAABGB |
| 42 | C_{Me} 6 | GAABBCAAAAGBGBCGGBGABBBGCCBCGBC_{Me} GCBCCAABGB |
| 43 | C_{Me} 7 | GAABBCAAAAGBGBCGGBGABBBGCCBCGBCGC MeBCCAABGB |
| 44 | C_{Me} 8 | GAABBCAAAAGBGBCGGBGABBBGCCBCGBCGC BC_{Me}CAABGB |
| 45 | C_{Me} 9 | GAABBCAAAAGBGBCGGBGABBBGCCBCGBCGC BCC_{Me}AABGB |

Then, the binding ability of the modified TfR aptamers to the mouse transferrin receptor proteins was measured in the same manner as in Example 2-1 above and the results are shown in Tables 6 to 9 below.

**Table 6**

| A→2'-O-Me | A_{Me} 1 | A_{Me} 2 | A_{Me} 3 | A_{Me} 4 | A_{Me} 5 | A_{Me} 6 | A_{Me} 7 | A_{Me} 8 | A_{Me} 9 |
|---|---|---|---|---|---|---|---|---|---|
| Bmax | 0.0158 | 0.0759 | 0.0341 | 0.0528 | 0.0431 | 0.0569 | 0.0271 | 0.028 | 0.0232 |
| K_{d} | 83.1525 | 54.6782 | 26.0605 | 20.9249 | 32.9936 | 43.6749 | 0.00151 | 37.2089 | 28.363 |

**Table 7**

| U→2'-O-Me | U_{Me} 1 | U_{Me} 2 | U_{Me} 3 | U_{Me} 4 | U_{Me} 5 | U_{Me} 6 | U_{Me} 7 |
|---|---|---|---|---|---|---|---|
| Bmax | 0.0335 | 0.0284 | 0.0253 | 0.0115 | 0.0264 | 0.00681 | 0.0604 |
| K_{d} | 42.6117 | 56.4206 | 0.2304 | 32.4054 | 63.6522 | 1.9E-13 | 1.8378 |

| U→2'-O-Me | U_{Me} 8 | U_{Me} 9 | U_{Me} 10 | U_{Me} 11 | U_{Me} 12 | U_{Me} 13 | |
|---|---|---|---|---|---|---|---|
| Bmax | 0.0635 | 0.0345 | 0.1198 | 0.0608 | 0.0578 | 0.0611 | |
| K_{d} | 72.4796 | 18.342 | 25.216 | 17.8343 | 28.0412 | 22.7857 | |

**Table 8**

| G→2'-O-Me | G_{Me} 1 | G_{Me} 2 | G_{Me} 3 | G_{Me} 4 | G_{Me} 5 | G_{Me} 6 | G_{Me} 7 | G_{Me} 8 | G_{Me} 9 | G_{Me} 10 |
|---|---|---|---|---|---|---|---|---|---|---|
| Bmax | 0.2525 | 0.1861 | 0.5331 | 0.3384 | 0.597 | 0.112 | 0.1102 | 0.0602 | 0.085 | 0.1037 |
| K_{d} | 26.315 2 | 9.6715 | 129.68 9 | 6.3271 | 75.368 6 | 3.50E-13 | 17.962 9 | 5.3864 | 27.500 9 | 29.954 2 |

**Table 9**

| C→2'-O-Me | C_{Me} 1 | C_{Me} 2 | C_{Me} 3 | C_{Me} 4 | C_{Me} 5 | C_{Me} 6 | C_{Me} 7 | C_{Me} 8 | C_{Me} 9 |
|---|---|---|---|---|---|---|---|---|---|
| Bmax | 0.1844 | 0.0978 | 0.0628 | 0.1453 | 0.0529 | 0.0693 | 0.0556 | 0.0662 | 0.1232 |
| K_{d} | 17.7309 | 11.4456 | 11.1241 | 6.9362 | 14.941 | 13.2319 | 0.3631 | 8.4617 | 7.66 |

Based on the results of the binding ability to the mouse transferrin receptor proteins shown in Tables 6 to 9, regions affecting reduction in K_{d} are shown in Tables 10 and 11 below, and 4 types of TfR combi aptamers chemically optimized at regions other than the above-described regions are shown in Table 12 below. In each of the sequences of Table 12 below, the 5'-terminus was labeled with Cy5 and idT was bound to the 3'-terminus.

**Table 10**

| mTfR protein | | | | | | | | | | | |
|---|---|---|---|---|---|---|---|---|---|---|---|
| QPCR | 5' | 1 | 2 | 3 | 4 | 5 | 6 | 7 | 8 | 9 | 10 |
| Original sequence | | G | A | A | n | n | C | A | A | A | A |
| 2'-O-Me U | | | | | 0.13 | 0.10 | | | | | |
| 2'-O-Me G | | 0.05 | | | | | | | | | |
| 2'-O-Me C | | | | | | | 0.07 | | | | |
| 2'-O-Me A | | | 0.01 | 0.01 | | | | 0.02 | 0.03 | 0.02 | 0.02 |

| mTfR protein | | | | | | | | | | | |
|---|---|---|---|---|---|---|---|---|---|---|---|
| QPCR | 11 | 12 | 13 | 14 | 15 | 16 | 17 | 18 | 19 | 20 | 21 |
| Original sequence | G | n | G | n | C | G | G | n | G | A | n |
| 2'-O-Me U | | n/a | | 0.17 | | | | 0.09 | | | n/a |
| 2'-O-Me G | 0.14 | | 0.01 | | | 0.21 | 0.02 | | n/a | | |
| 2'-O-Me C | | | | | 0.11 | | | | | | |
| 2'-O-Me A | | | | | | | | | | n/a | |

**Table 11**

| mTfR protein | | | | | | | | | | | | |
|---|---|---|---|---|---|---|---|---|---|---|---|---|
| QPCR | 22 | 23 | 24 | 25 | 26 | 27 | 28 | 29 | 30 | 31 | 32 | 33 |
| Original sequence | n | n | G | C | C | n | C | G | n | C | G | C |
| 2'-O-Me U | 2.51 | 0.06 | | | | 0.25 | | | 0.18 | | | |
| 2'-O-Me G | | | 0.07 | | | | | 0.25 | | | 0.05 | |
| 2'-O-Me C | | | | 0.11 | 0.18 | | 0.08 | | | 0.09 | | 3.36 |
| 2'-O-Me A | | | | | | | | | | | | |

| mTfR protein | | | | | | | | | | | | |
|---|---|---|---|---|---|---|---|---|---|---|---|---|
| QPCR | 34 | 35 | 36 | 37 | 38 | 39 | | 40 | 41 | 3' | | |
| Original sequence | n | C | C | A | A | n | | G | n | idT | | |
| 2'-O-Me U | 0.26 | | | | | 0.16 | | | 0.20 | | | |
| 2'-O-Me G | | | | | | | | 0.04 | | | | |
| 2'-O-Me C | | 0.14 | 0.16 | | | | | | | | | |
| 2'-O-Me A | | | | 0.02 | 0.03 | | | | | | | |

**Table 12**

| SEQ ID NO: | Name | Sequence (5'→3') |
|---|---|---|
| 46 | TfR 41mer_combi 1 | GAA BBC_{Me} AAA AG_{Me}B GBC G_{Me}GB GAB BBG CCB CG_{Me}B CGC BC_{Me}C_{Me} AAB GB |
| 47 | TfR 41mer_combi 2 | GAA BBC_{Me} AAA AGB GBC GGB GAB BBG CCB CGB CGC BC_{Me}C_{Me} AAB GB |
| 48 | TfR 41mer_combi 3 | GAA BBC AAA AG_{Me}B GBC G_{Me}GB GAB BBG CCB CG_{Me}B CGC BCC AAB GB |
| 49 | TfR 41mer_combi 4 | GAA BBC AAA AGB GBC GMeGB GAB UMeBG CCMeB CGMeB CGC BCMeCMe AAB GB |

### Example 8. Identification of Transferrin Receptor Protein Binding Ability and Receptor-mediated Endocytosis of Chemically Optimized TfR Aptamer

### 8-1. Transferrin Receptor Protein Binding Ability of Chemically Optimized TfR Aptamer

Transferrin receptor protein binding ability of the 4 types of chemically optimized TfR combi aptamers prepared in Example 7 (Table 12) was identified in the same manner as in Example 2-1 above.

As a result, as shown in FIG. 13, it was confirmed that the transferrin receptor protein binding ability of the chemically optimized TfR aptamers was improved compared to the TfR aptamer that was not chemically optimized (TfR-01-01-41) as a control.

### 8-2. Receptor-mediated Endocytosis of Chemically Optimized TfR Aptamer

Receptor-mediated endocytosis of the 4 types of chemically optimized TfR combi aptamers prepared in Example 7 (Table 12) was identified in the same manner as in Example 2-2 above using human HepG2 cells, mouse bEND.3 cells, or monkey Cos7 cells.

As a result, as shown in FIG. 14, it was confirmed that there was no difference in the receptor-mediated endocytosis of the human, mouse, and monkey cell lines between the TfR aptamer that is not chemically optimized (TfR-01-01-41) and the chemically optimized TfR aptamers.

### Example 9. Identification of Serum Stability of Chemically Optimized TfR Aptamer

Serum stability of the chemically optimized TfR combi aptamers prepared in Example 7 was identified.

Specifically, 45 µL of 100% serum was treated with 10 µL of the TfR 41mer_combi 1 (SEQ ID NO: 46) aptamer having a concentration of 10 µM and incubated at 37°C for 0, 4, 24, 48, and 72 hours. After the incubation was terminated, the resultant was treated with 5 µL of the TfR aptamer (TfR-01-01-41), which was not chemically optimized, had a concentration of 10 µM, and was used as a control, and diluted with 165 µL of distilled water. A mixture of phenol, chloroform, and isoamyl alcohol, mixed in a ratio of 25:24:1 [v/v] was added, in an equal volume, to the samples and mixed using a vortex mixer for 20 seconds. The mixture was centrifuged at room temperature at 16,000×*g* for 10 minutes, and a supernatant was transferred to a new tube. 5 sample buffers were added to the supernatant and heated at 95°C for 5 minutes. The samples were added to Urea PAGE and electrophoreses at 220 V for 25 minutes, and the gel was stained with SYBR gold, followed by gel image analysis using a Gel Doc^{™} EZ system infrared imaging system.

As a result, as shown in FIG. 15, it was confirmed that the chemically optimized TfR aptamer had improved serum stability, due to increased half-life, compared to the TfR aptamer that was not chemically optimized as the control.

### Example 10. Identification of Receptor-mediated Endocytosis of Chemically Optimized Antibody-conjugated TfR Aptamer

After the chemically optimized TfR combi aptamer (TfR 41mer_combi 1, SEQ ID NO: 46) prepared in Example 7 was conjugated with Rituximab, receptor-mediated endocytosis thereof was identified in the same manner as in Example 2-2.

As a result, as shown in FIG. 16, it was confirmed that the chemically optimized TfR aptamer, in the case of being conjugated with the antibody (Rituximab), efficiently transported the antibody via cell membrane of human cells.

### Example 11. Transferrin Receptor Protein Binding Ability of TfR Aptamer According to pH Condition

In order to identify whether the transferrin receptor protein binding ability of the TfR combi aptamer (TfR 41mer_combi 1, SEQ ID NO: 46) prepared in Example 7 varies according to pH conditions, the transferrin receptor protein binding ability of the TfR combi aptamer (TfR 41mer_combi 1, SEQ ID NO: 46) according to the pH conditions was identified using a 1X SB18 buffer having a pH of 7.5, 6.5, and 5.5 in the same manner as in Example 2-1.

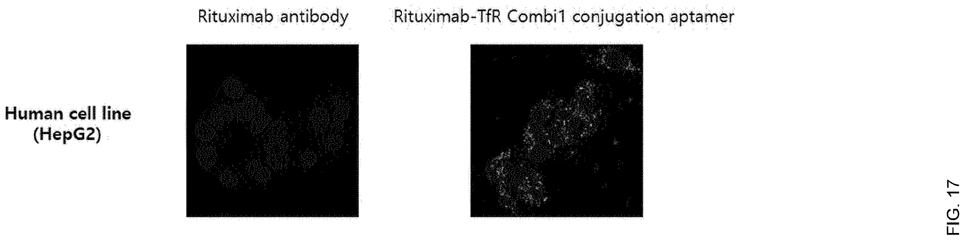

As a result, as shown in FIG. 17, it was confirmed that the TfR combi aptamer lost the binding ability to the transferrin receptor protein at an endosome pH, i.e., a pH of 5.5 to 6.5.

Based on the results of the above-described examples, the TfR aptamer of the present invention may be applicable as a drug carrier for nervous system diseases, particularly, brain and central nervous system diseases, as the TfR aptamer selectively targets the transferrin receptor proteins only, rapidly passes through the BBB by means of receptor-mediated endocytosis, may efficiently transport and deliver various therapeutic agents such as proteins, antibodies, nucleic acids, and low-molecular weight compounds through simple chemical bonds, exhibits interspecific cross-reactivity in transferrin receptors, and is easily chemically modified.

The above description of the present invention is provided for the purpose of illustration, and it would be understood by those skilled in the art that various changes and modifications may be made without changing technical conception and essential features of the present invention. Thus, it is clear that the above-described embodiments are illustrative in all aspects and do not limit the present invention. Furthermore, the scope of the present disclosure should be defined by the appended claims rather than the detailed description, and it should be understood that all modifications or variations derived from the meanings and scope of the present disclosure and equivalents thereof are included in the scope of the present disclosure.

## Claims

1. A transferrin receptor protein-specific aptamer comprising a nucleotide sequence of General Formula 1 below:
General Formula 1
AGTGTCGGTGATTTGCCTCGTCGCT (SEQ ID NO: 52).

2. The aptamer according to claim 1, wherein the aptamer further comprises 1 to 30 polynucleotides at one or more of the 5'-terminus and the 3'-terminus thereof.

3. The aptamer according to claim 1, wherein the aptamer comprises 30 to 150 polynucleotides.

4. The aptamer according to claim 1, wherein the aptamer comprises at least one polynucleotide sequence selected from the group consisting of SEQ ID NOS: 1 to 4 or a polynucleotide sequence having at least 90% homology therewith.

5. The aptamer according to claim 1, wherein the aptamer comprises at least one modification selected from the following modifications i) to iii) at one or more nucleotides constituting a polynucleotide sequence thereof:
i) 5^{th} carbon of thymine (T) of at least one nucleotide is substituted with at least one selected from the group consisting of a benzyl group, a naphthyl group, a 2-naphthyl group, a pyrrolebenzyl group, and tryptophan;
ii) -OH group of the 2^{nd} carbon of at least one nucleotide is substituted with at least one selected from the group consisting of a deoxy group, a methoxy group, an amino group, and fluorine (F); and
iii) at least one nucleotide is substituted with at least one selected from the group consisting of methylphosphonate, phosphorothioate, locked nucleic acid (LNA), peptide nucleic acid (PNA), and hexitol nucleic acid (HNA).

6. The aptamer according to claim 5, wherein the aptamer comprises at least one polynucleotide sequence selected from the group consisting of SEQ ID NOS: 5 to 49 or a polynucleotide sequence having at least 90% homology therewith.

7. The aptamer according to claim 1, wherein the aptamer is conjugated with at least one selected from the group consisting of polyethylene glycol (PEG), hexaethylene glycol (HEG), inverted deoxythymidine (idT), biotin, a fluorescent substance, an amine linker, a thiol linker, cholesterol, and a fatty acid at one or more of the 5'-terminus and the 3'-terminus of the nucleotide sequence thereof.

8. A pharmaceutical composition for preventing or treating a nervous system disease comprising the aptamer according to any one of claims 1 to 7 as an active ingredient.

9. The pharmaceutical composition according to claim 8, wherein the aptamer is further conjugated with at least one selected from the group consisting of a protein, an antibody, a nucleic acid, and a low-molecular weight compound.

10. The pharmaceutical composition according to claim 8, wherein the nervous system disease is a central nervous system disease.

11. The pharmaceutical composition according to claim 8, wherein the central nervous system disease comprises at least one selected from the group consisting of brain cancer, brain infection, dementia, Parkinson's disease, Alzheimer's disease, Pick's disease, Huntington's disease, multiple sclerosis, epilepsy, stroke, cerebral apoplexy, ischemic brain disease, memory loss, traumatic central nervous system disease, spinal cord injury disease, behavioral disorder, developmental disability, mental retardation, Hunter's disease, amyotrophic lateral sclerosis, Down syndrome, and schizophrenia.

12. A drug carrier comprising the aptamer according to any one of claims 1 to 7.

13. The drug carrier according to claim 12, wherein the drug carrier further comprises at least one selected from the group consisting of a protein, an antibody, a nucleic acid, and a low-molecular weight compound.
